# EUROPEAN PATENT SPECIFICATION

(11) **EP 1 077 256 B1**
(45) Date of publication and mention of the grant of the patent: **21.02.2007**
(21) Application number: 99919545.6
(22) Date of filing: 12.05.1999
(51) Int. Cl.: C12N 9/78, C12N 15/55, C12N 1/21, C12N 1/15

(54) **NOVEL GLUTAMINASE, GENE THEREOF AND PROCESS FOR PRODUCING THE SAME**
NEUE GLUTAMINASE UND ENTSPRECHENDES GEN, SOWIE VERFAHREN ZU DEREN HERSTELLUNG
NOUVELLE GLUTAMINASE, SON GENE ET SON PROCEDE DE PREPARATION

(30) Priority: 15.05.1998 JP 13408098; 11.09.1998 JP 25897498; 14.10.1998 JP 29244398; 30.03.1999 JP 8915799
(43) Date of publication of application: 21.02.2001
(73) Proprietor: Ajinomoto Co., Inc., Tokyo 104-0031 (JP)
(72) Inventor: KOIBUCHI, Kyoko, Ajinomoto Co. Inc., Kawasaki-shi, Kanagawa 210-0801 (JP); NAGASAKI, Hiroaki, Ajinomoto Co. Inc., Kawasaki-shi, Kanagawa 210-0801 (JP); YUASA, Ari, Ajinomoto Co. Inc., Kawasaki-shi, Kanagawa 210-0801 (JP); KATAOKA, Jiro, Ajinomoto Co. Inc., Kawasaki-shi, Kanagawa 210-0801 (JP); KITAMOTO, Katsuhiko, Ushiku-shi, Ibaraki 300-1232 (JP)
(74) Representative: Strehl Schübel-Hopf & Partner
(86) International application number: PCT/JP1999/002455
(87) International publication number: WO 1999/060104

(56) References cited:
- YANO T ET AL: "PRODUCTION AND LOCALIZATION OF ENZYMES ON SOFT GEL CULTIVATION" AGRICULTURAL AND BIOLOGICAL CHEMISTRY, vol. 55, no. 2, 1991, pages 387-392, XP001057982 ISSN: 0002-1369
- KOIBUCHI K ET AL: "Molecular cloning and characterization of a gene encoding glutaminase from Aspergillus oryzae." APPLIED MICROBIOLOGY AND BIOTECHNOLOGY, vol. 54, no. 1, July 2000 (2000-07), pages 59-68, XP002191327 ISSN: 0175-7598
- J. FERMENT. TECHNOL. TOSHIHIRO YANO ET AL: 'Purification and properties of glutaminase from Aspergillus Oryzae' vol. 66, no. 2, 1988, pages 137 - 143, XP002921814

## Description

### Technical Field

The present invention relates a novel glutaminase and a gene encoding the same. The glutaminase of the present invention can be utilized as an enzyme for food processing to convert glutamine into glutamic acid exhibiting stronger "umami" taste (umami).

### Background Art

For the production of soy sauce, miso, and other natural seasonings containing protein hydrolysate products, koji mould (filamentous fungus belonging to the genus Aspergillus) has been utilized. For example, soy sauce is produced through two process steps of koji-making and fermentation. In the koji-making step, the starting material is principally degraded by enzymes produced by koji mould. In such a process, it is important to increase the amount of glutamic acid among various tasteful materials in order to obtain stronger umami of soy sauce.

Glutamic acid is produced through two kinds of pathways. The first is the liberation of glutamic acid from protein caused by protease and peptidase. The second is generation of glutamic acid through hydrolysis of glutamine catalyzed by glutaminase (glutamine amidohydrolase).

In the production of soy sauce, liberation ratio of glutamic acid relative to its content in the starting material is not so high, and this is considered to be due to insufficient glutaminase activity of koji mould. Therefore, breeding of strains exhibiting high activities of protease and glutaminase through cell fusion of high protease activity strain and high glutaminase activity strain in solid koji has also been attempted (Ushijima, S. et al., Agric. Biol. Chem., 51 (4), 1051 (1987), Japanese Patent Publication (KOKOKU) No. Hei 3-73271/1992).

As for glutaminase, those derived from various bacteria and animals have been well investigated (Wakayama, M. et al., J. Ferment. Bioeng., 82, No.6, 592-597 (1996), Chung-Bok, Mi, et al., Biochem. J., 324, 193-200 (1997), Duran, S. et al., Biochem. Genet., 34, 453-465 (1996)). On the other hand, investigation about glutaminase of koji mould had been retarded, but extracellular glutaminase and intracellular glutaminase have been purified from one strain of Aspergillus oryzae, and they have been characterized (Yano, T. et al., J. Ferment. Technol., Vol. 66, No. 2, 137-143 (1988)). These glutaminases have a molecular weight of about 113,000, and substantially similar properties.

Further, there have been determined an amino acid sequence of N-terminal region of glutaminase derived from Aspergillus oryzae HG strain (Fukuoka Industrial Technology Center, Institute of Biology and Food, Research Summary of 1996 (199 )), and amino acid sequence within N-terminal region of glutaminase derived from Aspergillus oryzae (Food Research Institute, Aichi Prefectural Government, Japan, Annual Report of 1995 (Research Report) pp.3-4, (1996)) for purified glutaminases.

Meanwhile, because koji mould is excellent in the ability for secreting extracellular proteins, it has been attracted attention as a host for the production of recombinant proteins, and practically used for some enzymes.

### Disclosure of the Invention

As described above, koji mould has already afforded results as a material for genetic recombination technology, and its glutaminase has also been investigated to some extent. However, it cannot be considered to be fully investigated, and its further investigation has been desired. In addition, any genes encoding glutaminase of koji mould have not been isolated.

The present invention has been accomplished in view of the aforementioned state of the art, and its object is to provide a gene encoding glutaminase derived from koji mould.

The present inventors successfully purified glutaminase from Aspergillus oryzae, determined its partial amino acid sequence, and isolated DNA coding for the glutaminase based on the obtained information, and thus the present invention has been completed. Further, they also succeeded in isolating DNA encoding glutaminase of Aspergillus nidulans.

That is, the present invention provides the followings:
(1) a protein defined in any of the following (A) to (D):
   (A) a protein having an amino acid sequence represented by the amino acid numbers 1-670 of SEQ ID NO: 2;
   (B) a protein having an amino acid sequence represented by the amino acid numbers 1-669 of SEQ ID NO: 22;
   (C) a protein having an amino acid sequence represented by the amino acid numbers 1-670 of SEQ ID NO: 2 with substitution, deletion, insertion, addition or inversion of 1 to 170 amino acids, and having activity for catalyzing hydrolysis of glutamine to glutamic acid and ammonia;
   (D) a protein having an amino acid sequence represented by the amino acid numbers 1-669 of SEQ ID NO: 22 with substitution, deletion, insertion, addition or inversion of 1 to 170 amino acids, and having activity for catalyzing hydrolysis of glutamine to glutamic acid and ammonia;
(2) a DNA which encodes a protein defined in any of the following (A) to (D):
   (A) a protein having an amino acid sequence represented by the amino acid numbers 1-670 of SEQ ID NO: 2;
   (B) a protein having an amino acid sequence represented by the amino acid numbers 1-669 of SEQ ID NO: 22;
   (C) a protein having an amino acid sequence represented by the amino acid numbers 1-670 of SEQ ID NO: 2 with substitution, deletion, insertion, addition or inversion of 1 to 170 amino acids, and having activity for catalyzing hydrolysis of glutamine to glutamic acid and ammonia;
   (D) a protein having an amino acid sequence represented by the amino acid numbers 1-669 of SEQ ID NO: 22 with substitution, deletion, insertion, addition or inversion of 1 to 170 amino acids, and having activity for catalyzing hydrolysis of glutamine to glutamic acid and ammonia;
(3) the DNA of (2) which is a DNA defined in any of the following (a) to (d):
   (a) a DNA which contains nucleotide sequences represented by the nucleotide numbers 1174-1370, 1446-1741, 1800-2242, 2297-2880, 2932-3134, 3181-3324, 3380-3515, 3562-3628 of the nucleotide sequence of SEQ ID NO: 1 in this order;
   (b) a DNA which contains nucleotide sequences represented by the nucleotide numbers 1807-2000, 2061-2353, 2412-2854, 2915-3498, 3554-3756, 3806-3949, 3996-4131, 4180-4246 of the nucleotide sequence of SEQ ID NO: 21 in this order;
   (c) a DNA which hybridizes with the DNA of (a) under a stringent condition, and encodes a protein having activity for catalyzing hydrolysis of glutamine to glutamic acid and ammonia;
   (d) a DNA which hybridizes with the DNA of (b) under a stringent condition, and encodes a protein having activity for catalyzing hydrolysis of glutamine to glutamic acid and ammonia; wherein the stringent conditions comprise washing in 1 x SSC and 0.1 % SDS;
(4) the DNA of (2) which has a nucleotide sequence shown in SEQ ID NO: 1 or SEQ ID NO: 17;
(5) the DNA of (3) which has a nucleotide sequence shown in SEQ ID NO: 21 or SEQ ID NO: 25;
(6) a recombinant vector comprising the DNA of (2) inserted in a vector;
(7) a transformant of microorganism introduced with the DNA of (2) or with a recombinant vector according to (6);
(8) the transformant of (7) which is derived from a filamentous fungus or bacterium belonging to the genus Escherichia; and
(9) a method for producing glutaminase which comprises cultivating the transformant of (7) in a culture medium to produce glutaminase in the culture.

The term "glutaminase activity" used in this specification means activity for catalyzing hydrolysis of L-glutamine to L-glutamic acid and ammonia, and the activity may include activity for catalyzing hydrolysis of D-glutamine to D-glutamic acid and ammonia. The activity may also include activities for catalyzing hydrolysis of L-glutamine to L-glutamic acid and ammonia, and D-glutamine into D-glutamic acid and ammonia, and activity for catalyzing transfer reaction or hydrolysis reaction of glutamyl group of L-γ-glutamyl compounds. In the present specification two embodiments are disclosed as the glutaminase of the present invention. One or both of the embodiments, or equivalents thereof may occasionally be referred to as glutaminase of the present invention. Also, the DNA which encodes the glutaminase of the present invention may occasionally be referred to as glutaminase gene.

The glutaminase of the present invention is distinguished from known glutaminases derived from koji mould based on enzymological properties, and therefore it is considered a novel glutaminase.

The present invention will be explained in detail hereinafter.

### <1> Glutaminase of the present invention

The glutaminase of the present invention can be obtained from culture of Aspergillus oryzae RIB40 (ATCC 42149) by purifying it, for example, as follows.

Aspergillus oryzae RIB40 (ATCC 42149) is cultured with wheat bran, and the obtained bran koji is immersed in a buffer solution to prepare a crude enzyme extract. This crude enzyme extract is subjected to freeze and thawing, and insoluble fractions are removed to obtain a supernatant. This supernatant is subjected to ammonium sulfate fractionation to obtain a fraction not precipitated with 55% saturated ammonium sulfate but precipitated with 85% saturated ammonium sulfate. The ammonium sulfate is removed from this fraction, and resultant can further be fractionated by anion exchange chromatography, hydrophobic chromatography, and gel filtration chromatography to provide purified glutaminase. As resins for the chromatographies, there are exemplified DEAE-TOYOPEARL (Tosoh) for the anion exchange chromatography, Phenyl Sepharose (Pharmacia) for the hydrophobic chromatography, and Superdex (Pharmacia) for the gel filtration chromatography. These purification procedures may be repeatedly performed.

In each step for purification of glutaminase, the desired fraction is selected based on the glutaminase activity. The glutaminase activity can be determined by a modified version of the method of Hartman (Hartman, S.C., J. Biol. Chem., 243, 853-863 (1968), the hydroxamate method).

Enzymological properties of the glutaminase obtained from bran koji of Aspergillus oryzae RIB40 (ATCC 42149) as described above are shown in Table 1 together with enzymological properties of known glutaminases, one derived from of Aspergillus oryzae (Yano, T. et al., J. Ferment. Technol., Vol. 66, No.2, 137-143 (1988)), and one derived from Bacillus subtilis (Shimazu, H. et al., J. Brew. Soc. Japan, 86, No. 6, 441-446 (1991)).

**Table 1: Enzymological properties of glutaminase of the present invention and known glutaminases**

| | Glutaminase of The present invention | Derived from A. Oryzae Yano,T. et al. | Derived from B. subtilis Shimazu, H. et al. |
|---|---|---|---|
| Molecular weight | 82,090¹⁾ | 113,000²⁾ | 55,000 |
| Optimum pH | pH 9 | pH 9 | PH 6 |
| PH stability | pH 7 | pH 9 | PH 5-8 |
| Optimum temperature | 37-45°C | 45°C | 50°C |
| Temperature | 0-45°C | 0-37°C | 0-45°C |
| Stability | | | |
| Salt | 50% at 5% NaCl | 50% at 5% NaCl | 100% at 10% NaCl |
| Tolerance³⁾ | 20% at 18% NaCl | 10% at 18% NaCl | 85% or more at 25% NaCl |
| Substrate Specificity⁴⁾ | L-Gln (100%) D-Gln (106%) | L-Gln (100%) D-Gln (2%) | L-Gln (100%) D-Gln (67%) |
| | L-Asn (97%) | L-Asn (0%) | L-Asn (0%) |
| | D-Asn (104%) | D-Asn (0%) | D-Asn (0%) |
| Reaction Specificity | γ-Glu-p-NA | γ-Glu-p-NA | γ-Glu-p-NA (not tested) |
| Transfer⁵⁾ | (10%) | ( 0%) | ( 0%) |
| Hydrolysis⁶⁾ | (16%) | (131%) | ( 0%) |
| Km value | 1.24x10⁻³ M | 9.6x10⁻⁵ M | 6.4x10⁻⁴ M |

| | | | |
|---|---|---|---|
| 1) Measured by MALDI-TOFMS 2) Measured by gel filtration 3) Relative values based on the activity in the absence of NaCl that is defined as 100% 4) Relative values of activity for D-glutamic acid (D-Gln) based on the activity for L-glutamic acid (L-Gln) that is defined as 100% 5) L-γ-glutamyl-p-nitroanilide + GlyGlu → L-γ-glutamyl-GlyGlu + p-nitroanilide 6) L-γ-glutamyl-p-nitroanilide + H₂O → L-glutamate + p-nitroanilide | | | |

Based on the marked differences in enzymological properties shown above, in particular in the substrate specificity, the glutaminase of the present invention is concluded to be novel, and different from the known glutaminase derived from Aspergillus oryzae.

While the glutaminase of the present invention can be obtained by purifying it from culture of Aspergillus oryzae as described above, it can also be produced by expression of glutaminase gene of Aspergillus oryzae described below in a suitable host as will be described hereinafter.

As will be described hereinafter, the glutaminase derived from Aspergillus oryzae is expected to have the amino acid sequence represented by the amino acid numbers 1-670 in SEQ ID NO: 2 based on the nucleotide sequence of glutaminase gene. The molecular weight calculated from this amino acid sequence is about 76,000, and from its comparison with the value of molecular weight measured by MALDI-TOFMS, the glutaminase of the present invention is expected to be a glycoprotein.

The glutaminase of another embodiment of the present invention is derived from Aspergillus nidulans. The glutaminase derived from Aspergillus nidulans may be produced by purifying from a culture of Aspergillus nidulans in the same manner as described above, or expressing the glutaminase gene of Aspergillus nidulans in an appropriate host. The glutaminase derived from Aspergillus nidulans has deduced amino acid sequence represented by the amino acid numbers 1-669 of SEQ ID NO: 22 from the nucleotide sequence of the glutaminase gene.

As for the glutaminase of the present invention, so long as it has activity for catalyzing hydrolysis of glutamine to glutamic acid and ammonia, the aforementioned amino acid sequence may have substitution, deletion, insertion, addition or inversion of one or a plurality of amino acids.

The present invention also provides, as an embodiment of the glutaminase of the present invention, glutaminase of Aspergillus nidulans having the amino acid sequence shown in SEQ ID NO: 22. This glutaminase can be produced by purifying it from culture of Aspergillus nidulans in a manner similar to that described above, or by expression of glutaminase gene of Aspergillus nidulans in a suitable host.

### <2> DNA of the present invention

The DNA of the present invention can be obtained from genomic DNA of Aspergillus oryzae RIB40 (ATCC 42149), for example, as follows.

A partial amino acid sequence of the purified glutaminase is determined, and oligonucleotide primers for PCR (polymerase chain reaction) are synthesized based on the obtained information of the amino acid sequence to perform PCR using genomic DNA prepared from fungal cells of Aspergillus oryzae RIB40 (ATCC42149) as template. Partial sequences determined in the working examples of the present invention to be described hereinafter are shown in SEQ ID NOS: 3-10. Among these sequences, SEQ ID NO: 3 is an N-terminal amino acid sequence of the glutaminase protein, and the other sequences are internal amino acid sequences of the glutaminase. The amino acid sequences shown in SEQ ID NOS: 5 and 8 were not present in the amino acid sequence of glutaminase expected from the glutaminase gene. The third Ala and the ninth Thr in the amino acid sequence shown in SEQ ID NO: 7 were replaced by Thr and Ser respectively in the amino acid sequence of glutaminase expected from the glutaminase gene, and it was considered that they were reading errors in peptide sequencer..

The genomic DNA can be obtained by the method of Gomi (Gomi, K. et al., J. Gen. Appl. Microbiol., 35, 225 (1989)).

By using oligonucleotides having nucleotide sequences shown in SEQ ID NO: 11 and SEQ ID NO: 12 of Sequence Listing as the primers, a DNA fragment of about 230 bp can be obtained by the aforementioned PCR.

Then, plaque hybridization is performed for a genomic DNA library of Aspergillus oryzae RIB40 (ATCC 42149) utilizing λ phage as a vector by using the DNA fragment amplified by PCR as a DNA probe to obtain positive clones.

Within the cloned fragment obtained as described above, nucleotide sequence of a portion having a length of about 4 kb within a region having about 4.8 kb (XhoI fragment) is determined, and the result is shown in SEQ ID NO: 1 of Sequence Listing. In SEQ ID NO: 1, the amino acid sequence encoded by nucleotides of the nucleotide numbers 1234-1284 corresponds to the amino acid sequence of the amino acid numbers 1-17 in the N-terminal amino acid sequence of the glutaminase protein shown in SEQ ID NO: 3. The amino acid sequences shown in SEQ ID NOS: 4, 6, 7, 9 and 10 respectively correspond to the amino acid sequences encoded by nucleotides of the nucleotide numbers 2618-2647, 2762-2803, 2804-2848, 2957-2986, and 2576-2605 of the nucleotide sequence shown in SEQ ID NO: 1.

From the above, it is clear that DNA having the nucleotide sequence shown in SEQ ID NO: 1 is a glutaminase gene.

From the comparison of the nucleotide sequence shown in SEQ ID NO: 1 and the nucleotide sequence of glutaminase cDNA to be described hereinafter, it was found that the nucleotide sequence of SEQ ID NO: 1 contained 8 exons (nucleotide numbers 1174 or 1135-1370, 1446-1741, 1800-2242, 2297-2880, 2932-3134, 3181-3324, 3380-3515, and 3562-3628), and these exons encoded an amino acid sequence comprised of 690 residues. This amino acid sequence is shown in SEQ ID NOS: 1 and 2. From the comparison of this amino acid sequence and the amino acid sequence of the N-terminal of the glutaminase protein shown in SEQ ID NO: 3, it is estimated that the sequence of the amino acid numbers -20 to -1 is a signal peptide, and the sequence of the amino acid numbers 1-670 is the mature protein in SEQ ID NO: 2. While the initiation codon is estimated to be ATG of the nucleotide numbers 1174-1176, the possibility that it consists of ATG at the nucleotide numbers 1135-1138 cannot be denied.

From the above, it is strongly suggested that DNA having the nucleotide sequence shown in SEQ ID NO: 1 contains a promoter and a region encoding glutaminase (including signal peptide).

The DNA of the present invention may be DNA of the nucleotide sequence shown in SEQ ID NO: 1 of which introns are removed, i.e., DNA comprising nucleotide sequences of nucleotide numbers 1174-1370, 1446-1741, 1800-2242, 2297-2880, 2932-3134, 3181-3324, 3380-3515 and 3562-3628 in this order, so long as it encodes the glutaminase of the present invention. Such DNA can be obtained, for example, as cDNA of the aforementioned glutaminase gene.

Glutaminase cDNA can be obtained, for example, from a cDNA library prepared from poly(A) RNA of Aspergillus oryzae by hybridization which utilizes DNA having the nucleotide sequence of SEQ ID NO: 1 or a part thereof (e.g., the aforementioned probe of about 230 bp).

Glutaminase cDNA can also be obtained by PCR utilizing oligonucleotides having the nucleotide sequences of SEQ ID NOS: 13 and 14 as primers, and by 3'-RACE utilizing oligonucleotides having the nucleotide sequences of SEQ ID NOS: 15 and 16 as primers. An exemplary nucleotide sequence of cDNA obtained from a highly glutaminase productive strain of Aspergillus oryzae is shown in SEQ ID NO: 17 of Sequence Listing. The amino acid sequence deduced from this nucleotide sequence is shown in SEQ ID NOS: 17 and 18. When the nucleotide sequence of this cDNA was compared with the sequence of the coding region in the genomic gene obtained in Example 2, they were identical except that "C" at the nucleotide number 54 of the cDNA (SEQ ID NO: 17) was "G" (nucleotide number 1227) in the genomic gene (SEQ ID NO: 1). This difference between the nucleotide sequences of the cDNA and the genomic gene is estimated to be due to difference of gene sequence between the strains.

The DNA of the present invention may be any one encoding glutaminase, and it includes, in addition to DNA having the nucleotide sequence shown in SEQ ID NO: 1 or SEQ ID NO: 17, those DNA of which unnecessary portions in 5' region have been removed. Depending on purpose of the use, it may be one encoding only the mature protein. DNA of which one or more codons encoding amino acids in the coding region are replaced with equivalent codons encoding the same amino acids is included in the DNA of the present invention. Further, the DNA of the present invention may be one encoding glutaminase having substitution, deletion, insertion, addition or inversion of 1 to 170 amino acids at one or a plurality of sites, so long as the activity of glutaminase is not degraded.

As will be described hereinafter, the amino acid sequence of glutaminase of Aspergillus oryzae shown in SEQ ID NO: 2 and the amino acid sequence of glutaminase of Aspergillus nidulans shown in SEQ ID NO: 22 have about 73% of homology, and about 170 amino acid residues are different between them as for the mature protein portion.

DNA encoding a protein substantially the same as glutaminase such as those mentioned above can be obtained by modifying the nucleotide sequence of glutaminase gene, for example, by the site-specific mutagenesis so that amino acids should be substituted, deleted, inserted or added at a particular site. Such modified DNA as mentioned above may also be obtained by a conventionally known mutagenesis treatment. As such a mutagenesis treatment, there can be mentioned a method comprising treating DNA encoding glutaminase with hydroxylamine or the like in vitro, and a method comprising irradiating a bacterium belonging to the genus Escherichia with ultraviolet light, or treating it with a mutagenic agent conventionally utilized for mutagenesis such as N-methyl-N'-nitro-N-nitrosoguanidine (NTG), and nitrous acid.

The substitution, deletion, insertion, addition and inversion mentioned above include those due to difference among strains, and naturally occurring mutations.

DNA encoding a protein substantially the same as glutaminase can be selected by expressing DNA having mutations as described above in a suitable cell, and examining the expression product for glutaminase activity. DNA encoding a protein substantially the same as glutaminase can also be obtained by isolating DNA which hybridizes with DNA having any one of nucleotide sequences of nucleotide numbers 1174-1370, 1446-1741, 1800-2242, 2297-2880, 2932-3134, 3181-3324, 3380-3515, and 3562-3628 in the nucleotide sequence of SEQ ID NO: 1 in Sequence Listing, or DNA having the nucleotide sequence of the nucleotide numbers 1-2070 in the nucleotide sequence of SEQ ID NO: 17 under a stringent condition, and encodes a protein having the glutaminase activity. The term "stringent condition" herein used means a condition where hybridization is performed at a salt concentration corresponding to that of washing step of usual Southern hybridization, i.e., 1×SSC, 0.1% SDS, preferably 0.1×SSC, 0.1% SDS. Genes which hybridize under such a condition may also include those having a stop codon generated to interrupt the coding sequence, those having lost their activity due to mutation at the active center and the like, but they can easily be removed by ligating the genes to a commercially available active expression vector, and determining glutaminase activity by the method described hereinafter.

The DNA of the present invention can also be obtained from chromosome DNA or cDNA of microorganism of another species belonging to the genus Aspergillus, for example, Aspergillus nidulans. Specifically, it can be obtained from a chromosome DNA library of Aspergillus nidulans, for example, Aspergillus nidulans A26 strain by hybridization. A probe for the hybridization can be prepared by synthesizing oligonucleotide primers for PCR based on the aforementioned nucleotide sequence of the glutaminase gene of Aspergillus oryzae, and performing PCR using genome DNA prepared from cells of Aspergillus nidulans, e.g., Aspergillus nidulans A26 strain as template. As the primers for PCR, oligonucleotides having nucleotide sequences of SEQ ID NOS: 19 and 20 can be mentioned.

The nucleotide sequence and the amino acid sequence of the glutaminase gene of Aspergillus nidulans A26 obtained in the working examples to be described hereinafter in the manner described above are shown in SEQ ID NO: 21. The amino acid sequence is also shown in SEQ ID NO: 22. The homology between the glutaminase gene of Aspergillus nidulans and the glutaminase gene of Aspergillus oryzae was about 58% for the whole gene, about 68% for the coding region, and about 73% for the encoded amino acid sequence.

Glutaminase cDNA can also be obtained from a cDNA library prepared from poly(A) RNA of Aspergillus nidulans by, for example, PCR using oligonucleotides having nucleotide sequences SEQ ID NOS: 23 and 24. An exemplary nucleotide sequence of cDNA obtained from Aspergillus nidulans A26 is shown in SEQ ID NO: 25 of Sequence Listing. The amino acid sequence deduced from this nucleotide sequence is shown in SEQ ID NOS: 25 and 26.

The DNA of the present invention includes a DNA which encodes a protein having an amino acid sequence represented by the amino acid numbers 1-669 of SEQ ID NO: 22 in Sequence Listing with substitution, deletion, insertion, addition or inversion of one or a plurality of amino acids, and having activity for catalyzing hydrolysis of glutamine to glutamic acid and ammonia. The DNA of the present invention also includes a DNA which encodes a DNA which contains nucleotide sequences represented by the nucleotide numbers 1807-2000, 2061-2353, 2412-2854, 2915-3498, 3554-3756, 3806-3949, 3996-4131, 4180-4246 of the nucleotide sequence of SEQ ID NO: 21 in Sequence Listing in this order, and a DNA which hybridizes with the aforementioned DNA under a stringent condition, and encodes a protein having activity for catalyzing hydrolysis of glutamine to glutamic acid and ammonia.

The DNA of the present invention was obtained as described above in the wording examples to be described hereinafter. However, since its nucleotide sequence has been elucidated, it can easily be cloned by PCR, hybridization or the like from genomic DNA of Aspergillus oryzae RIB40 (ATCC 42149), Aspergillus nidulans A26, or other strains of Aspergillus oryzae and

### Aspergillus nidulans.

### <3> Use of the DNA of the present invention

The DNA of the present invention can be utilized for breeding of filamentous fungi such as koji mould or production of glutaminase. For example, glutaminase activity can be enhanced by intracellularly introducing the DNA of the present invention, preferably as its multiple copies, into filamentous fungus. Glutaminase can be produced by expressing the DNA of the present invention in a suitable host. A filamentous fungus such as koji mould and glutaminase obtained as described above can be utilized for the production of soy sauce, miso, and other seasonings containing protein hydrolysate products.

As the filamentous fungus to be introduced with the DNA of the present invention, there can be mentioned filamentous fungi belonging to the genus Aspergillus such as Aspergillus oryzae, Aspergillus niger and Aspergillus nidulans, those belonging to the genus Neurospora such as Neurospora crassa, those belonging to the genus Rhizomucor such as Rhizomucor miehei, and the like.

The vector for introducing the DNA of the present invention into filamentous fungi such as those mentioned above is not particularly limited, and those usually used for the breeding of filamentous fungi and the like can be used. As those used for Aspergillus oryzae, there can be mentioned, for example, pUNG (Lee, B.R. et al., Appl. Microbiol. Biotechnol., 44, 425-431 (1995)), pMARG (Tsuchiya, K. et al., Appl. Microbiol. Biotechnol., 40, 327-332 (1993)), pUSC (Gomi, K. et al., Agric. Biol. Chem. 51, 2549-2555 (1987)) and the like. pUNG contains a marker complementing niaD⁻ (nitrate assimilation ability defficiency) of Aspergillus oryzae niaD300 (Minetoki, T. et al., Curr. Genet. 30, 432-438 (1996)), pMARG contains a marker complementing argB⁻ (arginine auxotroph) of Aspergillus oryzae M2-3 (Gomi, K. et al., Agric. Biol. Chem., 51(9), 2549-2555 (1987)), and pUSC contains a marker complementing sC⁻ (ATP sulfurylase defficiency) of Aspergillus oryzae NS4 (Yamada, O. et al., Biosci. Biotech. Biochem., 61(8), 1367-1369 (1997)).

Among these vectors, pUNG and pMARG contain a promoter of glucoamylase gene (glaA) and α-amylase gene (terminator of amyB), and the DNA of the present invention (region of the nucleotide numbers 1136-4777 or 1177-4777 in SEQ ID NO: 1) can be expressed in them under the control of the promoter to produce glutaminase by inserting the DNA into them in the downstream of the promoter in such a manner that the frames should be conformed. When pUSC is used, because pUSC does not contain a promoter, expression of the gene of the present invention can be obtained by introducing a plasmid such as pUC19 inserted with the DNA of the present invention and pUSC into a host filamentous fungus through co-transformation of them. Since the nucleotide sequences of SEQ ID NO: 1 is likely to contain a promoter as described hereinbefore, it is considered that glutaminase can be expressed even if the DNA of the present invention is inserted into the aforementioned vector together with a promoter.

Those vectors, promoters and markers described in the literature mentioned below can also be used depending on the host filamentous fungus. In Table 2, promoters are indicated by the enzyme names encoded by corresponding genes.

**Table 2**

| Literature | Promoter | Marker | Host filamentous fungus |
|---|---|---|---|
| International Patent Application | Neutral α-amylase | | *Aspergillus niger* |
| Publication in Japanese (KOHYO) | | argB | *Aspergillus niger* |
| No. Hei 4-503450/1992 | | argB | *Aspergillus nidulans* |
| | | trpC | *Aspergillus nidulans* |
| | | amdS | *Aspergillus nidulans* |
| | | pyr4 | *Neurospora crassa* |
| | | DHFR | *Neurospora crassa* |
| Japanese Patent Unexamined | Taka-amylase | | *Aspergillus oryzae* |
| Publication (KOKAI) No. Sho 62-272988/1987 | Aspartic protease | | *Rhizomucor miehei* |
| | Lipase | | *Rhizomucor miehei* |
| | Glucoamylase, lipase Amylase, glucoamylase, cellulase Protease, glycolytic pathway enzymes | | *Aspergillus niger* |
| Japanese Patent Unexamined Publication No. Hei 7-51067/1995 | Taka-amylase | | *Genus Aspergillus* |
| Japanese Patent Unexamined Publication No. Hei 7-115976/1995 | Novel promoter sequence is mentioned. | | *Aspergillus oryzae* |
| Japanese Patent Unexamined Publication No. Hei 7-59571/1995 | Novel promoter sequence is mentioned. | | *Aspergillus niger* |
| Journal of Japan Society for Bioscience, Biotechnology and Agrochemistry, | α-amylase (amyB) | | *Aspergillus oryzae* |
| Vol. 71, No. 10 (1997) 1018-1023 | Glucoamylase (glaA) | | *Aspergillus oryzae* |
| | Glucosidase (agdA) | | *Aspergillus oryzae* |

Transformation of filamentous fungi can be performed by the methods mentioned in the aforementioned literature as well as other known methods. Specifically, Aspergillus oryzae, for example, can be transformed as follows.

Fungal cells (conidiospores) are inoculated in DPY culture medium (2% glucose, 1% peptone, 0.5% yeast extract, pH 5.0), and cultured at 30°C for around 24 hours with vigorous shaking. The culture medium is filtered through Myracloth (CALBIO CHEM), sterilized gauze or the like to collect the fungal cells, the cells are washed with sterilized water, and moisture is sufficiently removed from the cells. The cells are transferred into a test tube, added with an enzyme solution (1.0% Yatalase (Takara Shuzo), or 0.5% Novozyme (Novo Nordisk) and 0.5% cellulase (e.g., Cellulase Onozuka, Yakult), 0.6 M (NH₄)₂SO₄, 50 mM malic acid, pH 5.5), and gently shaken at 30°C for around 3 hours. The degree of protoplastization is observed with a microscope, and they are stored on ice if they show good protoplastization.

The aforementioned enzymatic reaction mixture is filtered through Myracloth to remove the fungal cell residue, and the filtrate containing protoplasts is added with an equal volume of Buffer A (1.2 M sorbitol, 50 mM CaCl₂, 35 mM NaCl, 10 mM Tris-HCl, pH 7.5), and placed on ice. The mixture is centrifuged at 0°C and 2,500 rpm for 8 minutes, and gently stopped, and the pellet is washed with Buffer A, and suspended in an optimum volume of Buffer A.

A DNA solution of not more than 20 µl (5-10 µg) is added to 100-200 µl of the protoplast suspension, and placed on ice for 20-30 minutes. To the mixture, 250 µl of Buffer B (polyethylene glycol 6000, 50 mM CaCl₂, 10 mM Tris-HCl, pH 7.5) is added and gently mixed, again 250 µl of Buffer B is added and gently mixed, further 850 µl of Buffer B is added and gently mixed, and then the mixture is left stand at room temperature for 20 minutes. Then, 10 ml of Buffer A is added to the mixture, and the test tube is inverted and subjected to centrifugation at 0°C and 2,000 rpm for 8 minutes. Subsequently, the pellet is suspended in 500 µl of Buffer A.

A suitable amount of the above suspension is added to 5 ml top agar, which has been divided into fractions and warmed beforehand, overlaid on an under layer culture medium (selection medium containing 1.2 M sorbitol, which is prepared depending on the kind of marker), and cultured at 30°C. Grown fungal cells are transferred on the selection medium, and confirmed to be transformants. Recombinant DNA is prepared from the fungal cells. It is preferable to confirm that the DNA of the present invention is introduced into the recombinant DNA by restriction enzyme analysis, Southern analysis or the like.

When the transformants obtained as described above are cultured under a condition suitable for the promoter used, the glutaminase gene is expressed, and thus glutaminase is produced.

By allowing a culture of transformants that are introduced with the gene of the present invention and have enhanced glutaminase activity to react with protein, protein hydrolysis products having higher sodium glutamate content and stronger umami can be afforded. Examples of the protein to be reacted with the culture include, for example, those of soybean, wheat, wheat gluten and the like, and it may be those of defatted soybean, or any one of various proteins subjected to food processing such as swelling and solubilization, or proteins isolated from these various kinds of materials.

As for the condition of the reaction of the culture of transformants with the protein, for example, a starting material having a concentration of 0.2-50% may be mixed with a culture of transformants in the presence of a proteolytic enzyme, and allowed to react at 5-60°C for 4 hours to 10 days.

After the completion of the reaction, insoluble unreacted proteins, fungal cells and the like can be removed by using conventional separation methods such as centrifugal separation or filtration. If required, the reaction mixture may be concentrated by vacuum concentration, reverse osmosis or the like, and the concentrate can be made into powder or granules by a drying process such as lyophilization, drying under reduced pressure, and spray drying. Thus, protein hydrolysates having high sodium glutamate content, and exhibiting stronger umami can be obtained without externally adding sodium glutamate.

### Description of Preferred Embodiments

The present invention will be explained more specifically with reference to the following examples hereinafter.

### Example 1: Purification of glutaminase from Aspergillus oryzae

Aspergillus oryzae RIB40 (ATCC 42149) strain was cultured with wheat bran, and glutaminase was purified from the culture. In the purification step, glutaminase activity was determined by a modified version of the method of Hartman (Hartman, S.C., J. Biol. Chem., 243, 853-863 (1968), the hydroxamate method). That is, to 125 µ of a solution containing 200 mM Tris-HCl (pH 7.0), 100 mM hydroxylamine hydrochloride, 50 mM L-glutamine, and 10 mM reduced glutathione, 25 µl of enzyme solution was added, and kept at 37°C for 1 hour. Then, the mixture was added with 125 µl of a solution composed of a mixture of equal volumes of 3 N hydrochloric acid, 12% trichloroacetic acid solution and 5% FeCl₃·6H₂O solution (dissolved in 0.1 N HCl), and absorption of the mixture at 525 nm was measured. As for the activity, the enzymatic activity which forms 1 µmol of L-glutamic acid γ-monohydroxamate at 37°C per minute was defined as 1 unit.

### (1) Cultivation

Wheat bran (Nisshin Flour Milling, 600 g), potassium phosphate (12 g), and distilled water (600 ml) were mixed well, introduced into six sets of deep Petri dish having a diameter of 15 cm in an amount of 160 g each, and autoclaved at 120°C for 20 minutes to prepare culture medium.

To a slant culture of Aspergillus oryzae RIB40 (ATCC 42149) sufficiently forming spores, sterilized water (5 ml) was poured, and stirred to prepare a spore suspension. The suspension was inoculated to the above culture medium. The culture medium inoculated with the spores was mixed well, and cultured at 30°C for 14 days. The culture medium was cared by stirring at 24 hours from the beginning of the cultivation.

### (2) Extraction of enzyme

The bran koji prepared as described above was immersed in three-fold volume of 20 mM potassium phosphate buffer (pH 7.4), 1 mM PMSF (phenylmethanesulfonyl fluoride), 0.1 mM EPNP (1,2-epoxy-3-(p-nitrophenyxy)propane), 1 mM EDTA, left stand at 4°C for 16 hours, and subjected to filtration through gauze and centrifugal separation (4°C, 7,500 rpm for 30 minutes) to afford a supernatant, which was used as a crude enzyme extract.

### (3) Fractionation by ammonium sulfate precipitation

The crude enzyme extract was frozen at -80°C, and gradually thawed at 4°C, and insoluble fractions were removed by filtration. The resultant was added with ammonium sulfate (1010 g/2, 880 ml) to afford a 55% saturated ammonium sulfate solution. The solution was stirred at 4°C for 4 hours, and centrifuged (4°C, 7,500 rpm, 30 minutes) to remove the precipitates. The supernatant was further added with ammonium sulfate (703 g /3,180 ml) to afford an 85% saturated ammonium sulfate solution. The solution was stirred at 4°C for 16 hours, and the produced precipitates were collected by centrifugation (4°C, 7,500 rpm, 30 minutes), and dissolved in 100 ml of 20 mM sodium phosphate buffer (pH 7.4). This was filtered through a filter having a pore diameter of 0.45 µm.

### (4) Desalination

5 ml from 100 ml of the filtrate obtained in the above (3) was loaded on a column for desalination which had been preliminarily equilibrated with 20 mM potassium phosphate buffer (pH 7.4), 100 mM NaCl beforehand (HiTrap Desalting, Pharmacia, 5 ml×5), and eluted with the same buffer solution. This desalination procedure was performed 20 times each for 5 ml divided from 100 ml of the filtrate. The active fractions were combined to amount 300 ml, and concentrated and desalted to 50 ml by ultrafiltration.

### (5) Anion exchange chromatography

The sample obtained above was adsorbed on a column filled with 250 ml DEAE-TOYOPEARL 650M (Tosoh), which had been preliminarily equilibrated with 20 mM potassium phosphate buffer (pH 7.4), and washed with the same buffer solution in a 4-fold volume of the column volume. After the washing, the column was eluted with linear gradient of NaCl increasing from 0 M to 0.5 M in the buffer of 8-fold volume of the column volume. The active fractions collected in the eluent (340 ml) was concentrated and desalted to 12 ml by ultrafiltration.

### (6) Hydrophobic chromatography

Then, fractionation by absorption chromatography was performed on a FPLC system (Pharmacia) utilizing HiLoad 26/10 Phenyl Sepharose High Performance (Pharmacia). This chromatographic procedure was performed twice for divided portions of the protein of DEAE-TOYOPEARL active fraction. 6 ml of the DEAE-TOYOPEARL active fraction added with 1 M ammonium sulfate was adsorbed on the column preliminarily equilibrated with 50 mM sodium phosphate buffer (pH 7.4), and 1 M ammonium sulfate, and the column was washed with the same buffer solution of 4-fold volume of the column volume. After the washing, the column was eluted with the buffer solution having linear ammonium sulfate gradient decreasing from 1 M to 0 M in 16-fold volume of the column volume. 300 ml of active fraction from the eluent was concentrated and desalted to 10 ml by ultrafiltration and centrifugal concentration (Centriprep 10, AMICON).

### (7) Gel filtration chromatography

The above sample was subjected to fractionation by gel filtration chromatography on a FPLC system utilizing HiLoad 26/60 Superdex 200pg (Pharmacia). This chromatographic procedure was performed 5 times for divided portions from the protein of the DEAE-TOYOPEARL active fraction. 2 ml of the active fraction obtained above was loaded on the column preliminarily equilibrated with 40 mM sodium phosphate buffer (pH 7.4), and 150 mM NaCl, and the column was eluted with the same buffer to collect active fraction. This gel filtration chromatography was repeated twice.

### (8) Results of purification

By the above-mentioned purification process, 500 µg of purified glutaminase was obtained. The molecular weight of the purified enzyme was determined by MALDI-TOFMS (Matrix assisted laser desorption ionization-time of flight mass spectrometer), and it was found to be 8,290. Total protein, total activity, specific activity, yield, and purity (based on the purity of the crude enzyme extract that was defined as 1) in various purification steps are summarized in Table 3.

**Table 3**

| Purification step | Glutaminase activity (Unit) | Amount of protein (mg) | Specific activity (unit/mg) | Yield (%) | Purity (fold) |
|---|---|---|---|---|---|
| Crude extract | 44.8 | 2528 | 0.018 | 100 | 1 |
| Freeze-thawing | 51 | 2230 | 0.023 | 114 | 1.28 |
| Ammonium sulfate precipitation | 16.2 | 665 | 0.024 | 36 | 1.33 |
| DEAE-TOYOPEARL | 7.2 | 186 | 0.039 | 16 | 2.2 |
| Phenyl Sepharose | 4.3 | 78.7 | 0.055 | 9.6 | 3.1 |
| First gel filtration | 0.8 | 1.1 | 0.73 | 1.6 | 39.4 |
| Second gel filtration | 0.4 | 0.5 | 0.88 | 1 | 48.9 |

### (9) Determination of partial amino acid sequence of glutaminase

The purified glutaminase (97 µg) in 500 mM Tris hydrochloric acid (pH 8.1), 6 M guanidine, and 2 mM EDTA was added with DTT (263 µg), substituted with nitrogen, and kept at 50°C for 3 hours. The reduction reaction was performed at room temperature overnight while the reaction mixture was shielded from light. The mixture was further added with iodoacetic acid (2900 µg), allowed to react at room temperature for 30 minutes, and desalted on a Sephadex G-25 (Pharmacia) column. The desalted sample was concentrated on centrifugal concentration machine VC-960 (TAITEC), and added with 50 mM ammonium hydrogencarbonate (pH 8.5) and 1 µg lysyl endopeptidase (SIGMA) to perform limited degradation at 37°C for 13 hours. After the reaction, the produced peptides were isolated by reversed-phase HPLC (Vydac Capillary C₁₈, Vydac), and each subjected to sequencing on a peptide sequencer PPSQ-10 (Shimazu Corporation) to determine internal partial amino acid sequences of glutaminase. Separately, the peptide not undergone the lysyl endopeptidase treatment was also subjected to sequencing on the peptide sequencer to determine its N-terminal amino acid sequence. The determined amino acid sequences are listed below.
N-terminal: ASTFSPARPPALPLAVK (SEQ ID NO: 3)
No. 52: Y(G/P) (N/V) (T/P) YAM (R/S) DI (SEQ ID NO: 4)
No. 55: VQY(T/G)EYDXY (SEQ ID NO: 5)
No. 59: DNDYLSQHYPILNK (SEQ ID NO: 6)
No. 67: WTAYLVEDTIYPANQ (SEQ ID NO: 7)
No. 62.5: VLLQSAIEGH (SEQ ID NO: 8)
No. 63: GIIGIQAMAV (SEQ ID NO: 9)
No. 62: XILKFXYXXQ (SEQ ID NO: 10)

In the aforementioned sequences, "X" represents an indefinite amino acid, and "/" means that the corresponding amino acid is one indicated before or after it.

### Example 2: Cloning of glutaminase gene of Aspergillus oryzae

The glutaminase gene was isolated from a genomic library of Aspergillus oryzae by plaque hybridization.

### (1) Production of probe by PCR

From cells of Aspergillus oryzae RIB40, genome DNA was prepared as follows according to the method of Gomi (Gomi, K. et al., J. Gen. Appl. Microbiol., 35, 225 (1989)).

Spores of Aspergillus oryzae RIB40 from two slant culture tubes were suspended in 0.85% NaCl, inoculated in 1 L of YPD culture medium (2% glucose, 1% peptone, 0.5% yeast extract, pH 5.0), and cultured at 30°C for 24 hours. The fungal cells were collected by filtration through gauze, immediately frozen with liquid nitrogen, and disrupted in a homogenizer (18000 rpm, 15 minutes) while cooling with liquid nitrogen or in a mortar. The resultant was added with 100 ml of 50 mM EDTA, 0.5% SDS, pH 8.0, and proteinase K to a final concentration of 0.1 mg/ml, and incubated at 50°C for 4 hours. This solution was subjected to phenol treatment, phenol/chloroform treatment, and chloroform treatment twice each. These treatments with organic solvents were performed by gently mixing the solution and the organic solvents, and separating an aqueous layer.

The solution from which the protein had been removed as described above was added with 1/10 volume of 3 M sodium acetate (pH 5.2) and 2.5-fold volume of ethanol, left at -20°C overnight, and centrifuged at 0°C and 10,000 rpm for 20 minutes. The resulting precipitates were rinsed, and carefully dissolved in TE buffer. This solution was added with RNase A (10 µg/ml), and incubated at 37°C for 30 minutes to degrade RNA. Then, the solution was mixed with 1/2 volume of phenol, left at 37°C for 10 minutes, mixed with 1/2 volume of chloroform, and centrifuged at 0°C and 10,000 rpm for 20 minutes to separate the aqueous layer. This aqueous layer was mixed with diethyl ether, and centrifuged at 0°C and 10,000 rpm for 5 minutes, and the diethyl ether layer was removed to eliminate the phenol remained in the solution. The resulting aqueous layer was added with 3 M sodium acetate solution (500 µl) and ethanol (5 ml), left at -80°C for one hour, and centrifuged at 10,000 rpm for 20 minutes to afford precipitates. These precipitates were rinsed, dissolved in TE buffer (5 ml), added with a small amount of chloroform, and stored under refrigeration.

After PCR using the genome DNA obtained as described above as template, and oligonucleotides which were synthesized based on the partial amino acid sequences (No. 52 and No. 67) and had the following sequences as primers, a partial sequence of glutaminase gene was obtained, and this was used as a probe for hybridization. The sequences of these primers were designed by referring to the codon frequency of Aspergillus nidulans (Andrew, T. Lloyd et al., Mol. Gen. Genet., 230, 288-294 (1991)). (5' end primer)
TAC CCC AAC ACC TAT GCT ATG CGC GAT ATC (SEQ ID NO: 11) (3' end primer)
TTG GTT CGC CGG ATA AAT AGT ATC TTC GAC CAA GTA (SEQ ID NO: 12)

The PCR reaction was performed by heat-denaturation at 95°C for 9 minutes, and a cycle of 94°C for 1 minute, 53°C for 1 minute, and 72°C for 1.5 minutes, which cycle was repeated for 35 cycles. This provided an about 230 b glutaminase gene fragment.

### (2) Screening of Aspergillus oryzae genomic library

Screening of Aspergillus oryzae genomic library was performed by using the gene fragment obtained in the above PCR reaction as a probe.

The Aspergillus oryzae genome DNA was digested with BamHI into about 10 kb fragments, and they were inserted into BamHI site of λ phage vectors (λ DASH II, STRATAGENE). The obtained recombinant DNAs were in vitro packaged to construct a λ phage library.

Using the aforementioned λ phage library, about 5,000 plaques were formed per one plate having a diameter of 15 cm. Ten plates on which plaques were formed were prepared, and each plate was blotted to two pieces of nylon membranes (Hybond-N+, Amersham). That is, the nylon membranes were placed on the plate for 3 minutes for the first piece, or for 5 minutes for the second piece, and then placed on filter paper sheets soaked with a denaturation solution (1.5 M NaCl, 0.5 M NaOH) for 7 minutes. Then, the membranes were placed on filter paper sheets soaked with a neutralization solution (1.5 M NaCl, 0.5M Tris-HCl (pH 7.4)) for 3 minutes, and then on other filter paper sheets soaked with the same buffer for 3 minutes for neutralization, left stand in 2×SSC for 3 minutes, and air-dried. After the drying, the membranes were placed on filter paper sheets soaked with 0.4 M NaOH for 20 minutes, shaken in 5×SSC for 1 minute, and air-dried.

Each of the nylon membranes mentioned above was immersed into a pre-hybridization buffer (50% formamide, 5×Denhardt's solution, 5×SSPE, 0.5% SDS), and kept at 42°C for 2 hours. The probe obtained in the PCR was labeled with [³²P-γ]-CTP using Random Label Kit (BOEHRINGER MANNHEIM). The labeled probe was kept at 100°C for 3 minutes, immediately transferred on ice so that the DNA double strands should be denatured, and added to the pre-hybridization buffer, and the hybridization was performed at 42°C overnight.

The aforementioned nylon membrane was subjected to washing in 2×SSC, 0.1% SDS at 65°C for 15 minutes twice, washing in 1×SSC, 0.1% SDS at 65°C for 15 minutes twice, and washing in 0.1×SSC, 0.1% SDS at 65°C for 15 minutes twice. After the washing, the membrane was brought into contact with an imaging plate (FUJIX), and detected with an image analyzer BAS2000 (FUJIX). As a result, 21 positive signals were detected from about 50,000 plaques.

The 21 positive plaques were collected, and subjected to the second screening. In the second screening, about 50 plaques were formed on a plate having a diameter of 10 cm, and hybridization was performed in the same manner as in the first screening. Four kinds of positive plaques were detected in the second screening. The third screening was performed in the same manner as in the second screening, and four kinds of positive clones were finally obtained.

Nucleotide sequencing of these four kinds of clones revealed that all of the clones contained the same sequence. The nucleotide sequence of XhoI fragment derived from one of the clones, and the amino acid sequence encoded by the nucleotide sequence are shown in SEQ ID NO: 1. The amino acid sequence alone is shown in SEQ ID NO: 2.

Escherichia coli DH5α strain which had been transformed with a plasmid obtained by inserting the aforementioned XhoI fragment into plasmid pBluescript was given a private number of AJ13495, and it was deposited at the National Institute of Bioscience and Human-Technology, Agency of Industrial Science and Technology, Ministry of International Trade and Industry (zip code: 305-8566, 1-3 Higashi 1-Chome, Tsukuba-shi, Ibaraki-ken, Japan) on September 9, 1998, and received an accession No. FERM BP-6490.

### Example 3: Cloning or glutaminase cDNA of Aspergillus oryzae

A highly glutaminase productive strain of Aspergillus oryzae was cultured in DPY culture medium (50 ml) at 30°C for 48 hours. The fungal cells were collected by filtration through gauze to obtain 1 g of cells. The cells were immediately frozen with liquid nitrogen, and disrupted in a mortar, from which 0.2 mg of total RNA were obtained according to the guanidine-cesium chloride ultracentrifugation method (Molecular Cloning, 2nd edition, Cold Spring Harbor Press (1989)). From the total RNA, mRNA was purified by using mRNA Purification Kit (Pharmacia), and cDNA was synthesized with cDNA PCR Library Kit (Takara Shuzo). By using this kit, a CA cassette adapter sequence is ligated to the 5' end of the obtained cDNA, and an oligo dT-RA sequence to the 3' end.

By using the cDNA obtained as described above as template, and oligonucleotides synthesized based on the nucleotide sequence of glutaminase genome and having the following sequences as primers, the glutaminase cDNA was amplified by PCR and 3'-RACE.
(5' end primer)
GAT CAT GAT GCA TTT CCT CTC GTT CTG TC (SEQ ID NO: 13)
(3' end primer)
GCA AAG TCA TCC GTA GAG ATC TGG TTC G (SEQ ID NO: 14)
(5' end primer for 3'-RACE)
GGC GAA CCA GAT CTC TAC GGA TGA CTT TGC (SEQ ID NO: 15)
(3' end primer for 3'-RACE)
CTG ATC TAG ACC TGC AGG CTC (SEQ ID NO: 16)

The PCR reactions were each performed by heat-denaturation at 95°C for 9 minutes, and a cycle of 94°C for 1 minute, 55°C for 1 minute, and 72°C for 1 minute, which cycle was repeated for 30 cycles. The PCR using the primers of SEQ ID NOS: 13 and 14 provided an about 1500 bp DNA fragment, and the 3'-RACE using the primers of SEQ ID NOS: 15 and 16 provided an about 780 bp DNA fragment.

The nucleotide sequence of these DNA fragments is shown in SEQ ID NO: 17. The amino acid sequence deduced from this nucleotide sequence is shown in SEQ ID NOS: 17 and 18.

### Example 4: Expression of glutaminase cDNA in Escherichia coli

Lac promoter, T7 promoter or Trp promoter is ligated to the upstream of the glutaminase cDNA, and inserted into a multi-cloning site of pBluescript (Stratagene). Escherichia coli DH5α is transformed with the obtained recombinant plasmid in a conventional manner, and the obtained transformants are selected on an agar medium containing 50 µg/ml of ampicillin. The selected transformants are cultured at 37°C in LB culture medium (1% trypton, 0.5% yeast extract, 1% NaCl) overnight. This culture medium (1 ml) is transferred into LB culture medium (50 ml), and cultured at 37°C. When its OD reaches 0.6 after 3 hours, IPTG (isopropyl-β-D-thiogalactopyranoside) is added to a final concentration of 1 mM to induce lac promoter, and the cultivation at 37°C is further continued for 4 hours. After the cultivation, the cells are collected, suspended in a buffer, and sonicated to provide protein inclusion bodies. These inclusion bodies are dissolved in a denaturant solution (8 M urea, 10 mM DTT, 50 mM NaCl, 50 mM Tris-HCl (pH 8.0), 5 mM EDTA), and insoluble fractions are removed by centrifugation. Refolding of the protein can be achieved by gradually lowering the urea concentration in the solution of solubilized protein.

Escherichia coli DH5α strain which had been transformed with a plasmid obtained by inserting the aforementioned glutaminase cDNA fragment into plasmid pBluescript was given a private number of AJ13496, and it was deposited at the National Institute of Bioscience and Human-Technology, Agency of Industrial Science and Technology, Ministry of International Trade and Industry (zip code: 305-8566, 1-3 Higashi 1-Chome, Tsukuba-shi, Ibaraki-ken, Japan) on September 9, 1998, and received an accession No. FERM BP-6491.

### Example 5: Cloning of glutaminase gene of Aspergillus nidulans

### (1) Production of probe by PCR

Based on the nucleotide sequence of the glutaminase gene of Aspergillus oryzae determined in Examples 1 and 2, oligonucleotide primers for PCR were synthesized, and PCR was performed by using genome DNA prepared from cells of Aspergillus nidulans A26 as template. The genome DNA was prepared in the same manner as in Example 2 according to the method of Gomi (Gomi, K. et al., J. Gen. Appl. Microbiol., 35, 225(1989)).

Oligonucleotides having sequences of the nucleotide numbers 1952-1979 and 2839-2868 of SEQ ID NO: 1 in Sequence Listing were synthesized as the PCR primers.
(5' end primer)
GAC GAC CAA GAT GGT CTG AGC TAC CAG T (1952-1979) (SEQ ID NO: 19)
(3' end primer)
GCA AAG TCA TCC GTA GAG ATC TGG TTC GCC (2839-2868) (SEQ ID NO: 20)

The PCR reaction was performed by heat-denaturation at 95°C for 3 minutes, and a cycle of 94°C for 1 minute, 37°C for 1 minute, and 72°C for 1 minute, which cycle was repeated for 30 cycles. This provided an about 900 b glutaminase gene fragment.

### (2) Screening of Aspergillus nidulans genomic library

Screening of Aspergillus nidulans genomic library was performed by using the gene fragment obtained in the above PCR reaction as a probe.

The Aspergillus nidulans genomic library was purchased from Fungal Genetics Strain Center (Kansas City, USA). Using this library, agar medium was prepared in a plate having a diameter of 10 cm, and a nylon membrane Hybond-N+ (Amersham) was overlaid thereon. The cells were inoculated on the nylon membrane to form about 50 colonies on one membrane. 30 pieces of plate on which the colonies were formed were prepared, and a nylon membrane was collected from each plate. Each nylon membrane was placed on a filter paper sheet soaked with a denaturation solution (1.5 M NaCl, 0.5 M NaOH) for 7 minutes. Then, the membrane was placed on a filter paper sheet soaked with a neutralization solution (1.5 M NaCl, 0.5M Tris-HCl (pH 7.4)) for 3 minutes, and then on another filter paper sheet soaked with the same buffer for 3 minutes for neutralization, left stand in 2×SSC for 3 minutes, and air-dried. After the drying, the membrane was placed on a filter paper sheet soaked with 0.4 M NaOH for 20 minutes, shaken in 5×SSC for 1 minute, and air-dried.

The nylon membrane mentioned above was soaked into a pre-hybridization buffer (50% formamide, 5×Denhardt's solution, 5×SSPE, 0.5% SDS), and kept at 65°C for 2 hours. The probe obtained in the PCR was labeled with [³²P-γ]-CTP using Random Label Kit (BOEHRINGER MANNHEIM). The labeled probe was kept at 100°C for 3 minutes, immediately transferred on ice so that the DNA double strands should be denatured, and added to the pre-hybridization buffer, and the hybridization was performed at 65°C overnight.

The aforementioned nylon membrane was subjected to washing in 2×SSC, 0.1% SDS at 65°C for 15 minutes twice, washing in 1×SSC, 0.1% SDS at 65°C for 15 minutes twice, and washing in 0.1×SSC, 0.1% SDS at 65°C for 15 minutes twice. After the washing, the membrane was brought into contact with an imaging plate (FUJIX), and detected with an image analyzer BAS2000 (FUJIX). As a result, 4 positive signals were detected from about 1500 colonies.

Nucleotide sequencing of these four kinds of clones revealed that all of the clones contained the same sequence. The nucleotide sequence of HindIII-EcoRV fragment derived from one of the clones, and the amino acid sequence encoded by the nucleotide sequence are shown in SEQ ID NO: 21. The amino acid sequence alone is shown in SEQ ID NO: 22.

Escherichia coli DH5α strain which had been transformed with a plasmid obtained by inserting the aforementioned HindIII-EcoRV fragment into plasmid pBluescript was given a private number of AJ13509, and it was deposited at the Fermentation Research Institute, Agency of Industrial Science and Technology, Ministry of International Trade and Industry (currently the National Institute of Bioscience and Human-Technology, Agency of Industrial Science and Technology, Ministry of International Trade and Industry) on September 22, 1998, and received an accession No. FERM BP-6520.

### Example 6 : Cloning of glutaminase cDNA of Aspergillus nidulans

Aspergillus nidulans A26 was cultured in 50 ml of YG culture medium (0.5% yeast extract, 2.5% glucose, 0.1% trace elements^{*}) at 37°C for 21 hours with shaking. The cells were collected on a filter paper sheet, and cultured in a plate containing minimal medium (0.6% NaNO₃, 0.152% KH₂PO₄, 0.052% KCl, 0.052% MgSO₄·7H₂O, 1% glucose, 0.1% trace elements^{*}, 2×10⁻⁵% biotin, 1.5% agar) at 37°C for 24 hours (trace elements^{*}: 0.1% FeSO₄· 7H₂O, 0.88% ZnSO₄·7H₂O, 0.04% CuSO₄·5H₂O, 0.015% MnSO₄·4H₂O, 0.01% Na₂B₄O₇·10H₂O, 0.005% (NH₄)₆Mo₇O₂₄·4H₂O).

The cells were collected from the plate, frozen with liquid nitrogen, and disrupted in a mortar. Total RNA was prepared using RNeasy Plant Mini Kit (QIAGEN) from the disrupted product, and mRNA was prepared from the total RNA using mRNA Purification Kit (Amersham Pharmacia Biotech). A cDNA library was prepared from the mRNA using cDNA Synthesis Kit and cDNA PCR Library Kit (Takara).

By using the cDNA library as template, and the following primers which had been designed based on the Aspergillus nidulans genomic DNA sequence, cloning of glutaminase cDNA was performed by PCR.
(5' end primer)
GCT TCA TAA TTC TCC TGT TGT TGA GTC (SEQ ID NO: 23)
Anti-sense primer
(3' end primer)
GGC TAT AAC TGA TGC TAT ATA CTA CCA CAC (SEQ ID NO: 24)

The reactions of the PCR were performed by heat denaturation at 94°C for 5 minutes, and 30 cycles of [94°C for 30 seconds, 55°C for 30 seconds, 72°C for 2 minutes]. As a result, an amplified fragment of about 2300 bp was observed, and thus a full length glutaminase cDNA was successfully obtained. The nucleotide sequence of this DNA fragment is shown in SEQ ID NO: 25. The amino acid sequence deduced from this nucleotide sequence is shown in SEQ ID NO: 26.

Escherichia coli DH5α strain which had been transformed with a plasmid obtained by inserting the aforementioned Aspergillus nidulans glutaminase cDNA fragment into TA cloning site of pGEM T Easy Vector (Promega) was given a private number of AJ13575, and it was deposited at the National Institute of Bioscience and Human-Technology, Agency of Industrial Science and Technology, Ministry of International Trade and Industry (zip code: 305-8566, 1-3 Higashi 1-Chome, Tsukuba-shi, Ibaraki-ken, Japan) on March 11, 1999, and received an accession No. FERM BP-6679.

### Example 7: Production of glutaminase

The glutaminase gene containing a promoter sequence was ligated to a vector containing a marker gene sC to provide a vector for transformation. Transformation was performed by using 10 µg of this plasmid DNA.

Conidiospores were inoculated in DPY culture medium, and cultured at 30°C for 24 hours with vigorous shaking. The culture medium was filtered through sterilized gauze, the cells were collected, and washed with sterilized water, and moisture was sufficiently removed from the cells. These cells were transferred to a test tube, added with an enzyme solution (20 ml, 1.0% Yatalase, Takara Shuzo), and gently shaken at 30°C for 3 hours. The degree of protoplastization was observed with a microscope, and they are stored on ice.

The aforementioned enzymatic reaction mixture was filtered through Myracloth to remove the cell residue, and the filtrate containing protoplasts was added with an equal volume of Buffer A (1.2 M sorbitol, 50 mM CaCl₂, 35 mM NaCl, 10 mM Tris-HCl, pH 7.5), and placed on ice. The mixture was centrifuged at 0°C and 1,500 rpm for 5 minutes, and gently stopped, and the pellet was washed with Buffer A twice, and suspended in 1 ml of Buffer A.

A DNA solution (10 µl, 10 µg) was added to 100 µl of the protoplast suspension, and placed on ice for 30 minutes. To the mixture, 250 µl of Buffer B (60% PEG (polyethylene glycol) 6000, 50 mM CaCl₂, 10 mM Tris-HCl, pH 7.5) was added and gently mixed, then 250 µl of Buffer B was added again and gently mixed, and 850 µl of buffer B was further added and gently mixed, and the mixture was left stand at room temperature for 20 minutes. Then, 10 ml of Buffer A was added to the mixture, and the test tube was inverted and subjected to centrifugation at 0°C and 1,500 rpm for 5 minutes. Then, the pellet was suspended in 500 µl of Buffer A.

The above suspension was added to 5 ml top agar culture medium, which had been divided into fractions and warmed beforehand, overlaid on M culture medium (1.2 M sorbitol, 0.2% ammonium chloride, 0.1% ammonium sulfate, 0.05% potassium chloride, 0.05% sodium chloride, 0.1% potassium dihydrogenphosphate, 0.05% magnesium sulfate heptahydrate, 0.002% ferrous sulfate, 2% glucose, pH 5.5), and cultured at 30°C. One strain of the grown cells was transferred on the M medium, and confirmed to be a transformant. Recombinant DNA was prepared from the cells, and it was confirmed that at least 2 copies of the DNA of the present invention existed by Southern analysis.

The transformant obtained above was cultured with wheat bran, and the glutaminase activity was measured for its extract. Wheat bran (Nisshin Flour Milling, 160 g),' potassium phosphate (3.2 g), and distilled water (160 ml) were mixed well, introduced into a deep Petri dish having a diameter of 15 cm, and autoclaved at 120°C for 20 minutes to prepare culture medium.

To a slant culture of the transformant sufficiently forming spores, sterilized water (10 ml) was poured, and stirred to prepare a spore suspension. The suspension was inoculated to the above culture medium. The culture medium inoculated with the spores was mixed well, and cultured at 30°C for 14 days. The culture medium was cared by stirring at 24 hours from the beginning of the cultivation.

The bran koji prepared as described above was immersed in three-fold volume of 20 mM potassium phosphate buffer (pH 7.4), 1 mM PMSF (phenylmethanesulfonyl fluoride), 0.1 mM EPNP (1,2-epoxy-3-(p-nitrophenyxy)propane), 1 mM EDTA, left stand at 4°C for 16 hours, and subjected to filtration through gauze and centrifugal separation (4°C, 15 minutes, 10,000 rpm) to provide a supernatant, which was used as a crude enzyme extract.

The glutaminase activity of this crude enzyme extract was determined. Another crude enzyme extract was similarly obtained from a transformant obtained by transforming a vector DNA having only the marker gene as control, and its glutaminase activity was measured.

**Table 4**

| | Glutaminase activity (per koji mg) |
|---|---|
| Transformant | 1.43 U/mg |
| Control strain | 0.38 U/mg |

As a result, marked activity increase was observed in the strain that had been introduced with the gene of the present invention, and it was demonstrated that the introduced glutaminase was expressed and produced.

### Industrial Applicability

The present invention provides a novel gene encoding glutaminase derived from koji mould. This gene can be used for breeding of koji mould, production of glutaminase, and production of seasonings such as soy sauce.

## Claims

1. A protein defined in any of the following (A) to (D) :
(A) a protein having an amino acid sequence represented by the amino acid numbers 1-670 of SEQ ID NO: 2;
(B) a protein having an amino acid sequence represented by the amino acid numbers 1-669 of SEQ ID NO: 22;
(C) a protein having an amino acid sequence represented by the amino acid numbers 1-670 of SEQ ID NO: 2 with substitution, deletion, insertion, addition or inversion of 1 to 170 amino acids, and having activity for catalysing hydrolysis of glutamine to glutamic acid and ammonia;
(D) a protein having an amino acid sequence represented by the amino acid numbers 1-669 of SEQ ID NO: 22 with substitution, deletion, insertion, addition or inversion of 1 to 170 amino acids, and having activity for catalysing hydrolysis of glutamine to glutamic acid and ammonia.

2. A DNA which encodes a protein defined in any of the following (A) to (D):
(A) a protein having an amino acid sequence represented by the amino acid numbers 1-670 of SEQ ID NO:2;
(B) a protein having an amino acid sequence represented by the amino acid numbers 1-669 of SEQ ID NO:22;
(C) a protein having an amino acid sequence represented by the amino acid numbers 1-670 of SEQ ID NO:2 with substitution, deletion, insertion, addition or inversion of 1 to 170 amino acids, and having activity for catalysing hydrolysis of glutamine to glutamic acid and ammonia;
(D) a protein having an amino acid sequence represented by the amino acid numbers 1-669 of SEQ ID NO: 22 with substitution, deletion, insertion, addition or inversion of 1 to 170 amino acids, and having activity for catalysing hydrolysis of glutamine to glutamic acid and ammonia.

3. The DNA according to claim 2 which is a DNA defined in any of the following (a) to (d):
(a) a DNA which contains nucleotide sequences represented by the nucleotide numbers 1174-1370, 1446-1741, 1800-2242, 2297-2880, 2932-3134, 3181-3324, 3380-3515, 3562-3628 of the nucleotide sequence of SEQ ID NO: 1 in this order;
(b) a DNA which contains nucleotide sequences represented by the nucleotide numbers 1807-2000, 2061-2353, 2412-2854, 2915-3498, 3554-3756, 3806-3949, 3996-4131, 4180-4246 of the nucleotide sequence of SEQ ID NO: 21 in this order;
(c) a DNA which hybridises with the DNA of (a) under a stringent condition, and encodes a protein having activity for catalysing hydrolysis of glutamine to glutamic acid and ammonia;
(d) a DNA which hybridises with the DNA of (b) under a stringent condition, and encodes a protein having activity for catalysing hydrolysis of glutamine, to glutamic acid and ammonia, wherein the stringent conditions comprise washing in 1xSSC and 0.1 % SDS.

4. The DNA according to claim 2 or 3 which has the nucleotide sequence shown in SEQ ID NO: 1 or SEQ ID NO: 17.

5. The DNA according to claim 2 or 3 which has a nucleotide sequence shown in SEQ ID NO: 21 or SEQ ID NO: 25.

6. A recombinant vector containing a DNA according to any of the claims 2 to 5.

7. A transformed microorganism in which is introduced the DNA according to any of the claims 2 to 5 or a recombinant vector according to claim 6.

8. The transformed microorganism according to claim 7 which is capable of producing glutaminase.

9. The transformed microorganism according to claim 7 or 8 which is derived from a filamentous fungus or bacterium belonging to the genus Escherichia.

10. A method for producing glutaminase which comprises cultivating the microorganism according to any of the claims 7 to 9 in a culture medium to produce glutaminase in the culture.

11. The use of a protein according to claim 1 for food processing.

12. The use of a microorganism according to any of the claims 7 to 9 for production of soy sauce, miso, or other seasonings containing protein hydrolysate products.

13. The protein according to claim 1 which is defined in the following (E) or (F):
(E) a protein having an amino acid sequence represented by the amino acid numbers 1-670 of SEQ ID NO: 2 with substitution, deletion, insertion, addition or inversion of 1 to 50 amino acids, and having activity for catalysing hydrolysis of glutamine to glutamic acid and ammonia;
(F) a protein having an amino acid sequence represented by the amino acid numbers 1-669 of SEQ ID NO: 22 with substitution, deletion, insertion, addition or inversion of 1 to 50 amino acids, and having activity for catalysing hydrolysis of gltuamine to glutamic acid and ammonia.

14. The DNA according to claim 2 which encodes a protein defined in the following (E) to (F):
(E) a protein having an amino acid sequence represented by the amino acid numbers 1-670 of SEQ ID NO: 2 with substitution, deletion, insertion, addition or inversion of 1 to 50 amino acids, and having activity for catalysing hydrolysis of glutamine to glutamic acid and ammonia;
(F) a protein having an amino acid sequence represented by the amino acid numbers 1-669 of SEQ ID NO: 22 with substitution, deletion, insertion, addition or inversion of 1 to 50 amino acids, and having activity for catalysing hydrolysis of glutamine to glutamic acid and ammonia.

15. The protein according to claim 13 which is defined in the following (G) or (H):
(G) a protein having an amino acid sequence represented by the amino acid numbers 1-670 of SEQ ID NO: 2 with substitution, deletion, insertion, addition or inversion of 1 to 10 amino acids, and having activity for catalysing hydrolysis of glutamine to glutamic acid and ammonia;
(H) a protein having an amino acid sequence represented by the amino acid numbers 1-669 of SEQ ID NO: 22 with substitution, deletion, insertion, addition or inversion of 1 to 10 amino acids, and having activity for catalysing hydrolysis of glutamine to glutamic acid and ammonia.

16. The DNA according to claim 14 which encodes a protein defined in the following (E) to (F):
(E) a protein having an amino acid sequence represented by the amino acid numbers 1-670 of SEQ ID NO: 2 with substitution, deletion, insertion, addition or inversion of 1 to 10 amino acids, and having activity for catalysing hydrolysis of glutamine to glutamic acid and ammonia;
(F) a protein having an amino acid sequence represented by the amino acid numbers 1-669 of SEQ ID NO: 22 with substitution, deletion, insertion, addition or inversion of 1 to 10 amino acids, and having activity for catalysing hydrolysis of glutamine to glutamic acid and ammonia.

## Patentansprüche

1. Protein, das durch einen der folgenden Punkte (A) bis (D) definiert ist:
(A) ein Protein mit einer Aminosäuresequenz der Aminosäurenummern 1-670 der SEQ ID NO: 2;
(B) ein Protein mit einer Aminosäuresequenz der Aminosäurenummern 1-669 der SEQ ID NO: 22;
(C) ein Protein mit einer Aminosäuresequenz der Aminosäurenummern 1-670 der SEQ ID NO: 2 mit Substitution, Deletion, Insertion, Addition oder Inversion von 1 bis 170 Aminosäuren und mit einer katalytischen Aktivität für die Hydrolyse von Glutamin zu Glutaminsäure und Ammoniak;
(D) ein Protein mit einer Aminosäuresequenz der Aminosäurenummern 1-669 der SEQ ID NO: 22 mit Substitution, Deletion, Insertion, Addition oder Inversion von 1 bis 170 Aminosäuren und mit einer katalytischen Aktivität für die Hydrolyse von Glutamin zu Glutaminsäure und Ammoniak.

2. DNA, welche für ein Protein kodiert, das durch einen der nachfolgenden Punkte (A) bis (D) definiert ist:
(A) ein Protein mit einer Aminosäuresequenz der Aminosäurenummern 1-670 der SEQ ID NO: 2;
(B) ein Protein mit einer Aminosäuresequenz der Aminosäurenummern 1-669 der SEQ ID NO: 22;
(C) ein Protein mit einer Aminosäuresequenz der Aminosäurenummern 1-670 der SEQ ID NO: 2 mit Substitution, Deletion, Insertion, Addition oder Inversion von 1 bis 170 Aminosäuren und mit einer katalytischen Aktivität für die Hydrolyse von Glutamin zu Glutaminsäure und Ammoniak;
(D) ein Protein mit einer Aminosäuresequenz der Aminosäurenummern 1-669 der SEQ ID NO: 22 mit Substitution, Deletion, Insertion, Addition oder Inversion von 1 bis 170 Aminosäuren und mit einer katalytischen Aktivität für die Hydrolyse von Glutamin zu Glutaminsäure und Ammoniak.

3. DNA nach Anspruch 2, wobei die DNA in einem der nachfolgenden Punkte (a) bis (d) definiert ist:
(a) eine DNA, die die Nucleotidsequenzen der Nucleotidnummern 1174-1370, 1446-1741, 1800-2242, 2297-2880, 2932-3134, 3181-3324, 3380-3515, 3562-3628 der Nucleotidsequenz der SEQ ID NO: 1 in dieser Reihenfolge enthält;
(b) eine DNA, die die Nucleotidsequenzen der Nucleotidnummern 1807-2000, 2061-2353, 2412-2854, 2915-3498, 3554-3756, 3806-3949, 3996-4131, 4180-4246 der Nucleotidsequenz der SEQ ID NO: 21 in dieser Reihenfolge enthält;
(c) eine DNA, die mit der DNA nach (a) unter einer stringenten Bedingung hybridisiert und für ein Protein mit einer katalytischen Aktivität für die Hydrolyse von Glutamin zu Glutaminsäure und Ammoniak kodiert;
(d) eine DNA, die mit der DNA nach (b) unter einer stringenten Bedingung hybridisiert und für ein Protein mit einer katalytischen Aktivität für die Hydrolyse von Glutamin zu Glutaminsäure und Ammoniak kodiert,
wobei die stringenten Bedingungen das Waschen in 1xSSC und 0,1 % SDS umfassen.

4. DNA nach Anspruch 2 oder 3, welche die in SEQ ID NO: 1 oder SEQ ID NO: 17 gezeigte Nucleotidsequenz hat.

5. DNA nach Anspruch 2 oder 3, welche die in SEQ ID NO: 21 oder SEQ ID NO: 25 gezeigte Nucleotidsequenz hat.

6. Rekombinanter Vektor, der eine DNA nach einem der Ansprüche 2 bis 5 enthält.

7. Transformierter Mikroorganismus, worin die DNA nach einem der Ansprüche 2 bis 5 oder ein rekombinanter Vektor nach Anspruch 6 eingeführt ist.

8. Transformierter Mikroorganismus nach Anspruch 7, der Glutaminase produzieren kann.

9. Transformierter Mikroorganismus nach Anspruch 7 oder 8, der von einem filamentösen Pilz oder einem Bacterium der Gattung Escherichia abgeleitet ist.

10. Verfahren zum Herstellen von Glutaminase, welches das Kultivieren des Mikroorganismus nach einem der Ansprüche 7 bis 9 in einem Kulturmedium umfaßt, wobei Glutaminase in der Kultur produziert wird.

11. Verwendung eines Proteins nach Anspruch 1 für die Nahrungsmittelverarbeitung.

12. Verwendung eines Mikroorganismus nach einem der Ansprüche 7 bis 9 für die Herstellung von Sojasauce, Miso oder anderer Würzstoffe, die Proteinhydrolysatprodukte enthalten.

13. Protein nach Anspruch 1, das in den folgenden Punkten (E) oder (F) definiert ist:
(E) ein Protein mit einer Aminosäuresequenz der Aminosäurenummern 1-670 der SEQ ID NO: 2 mit Substitution, Deletion, Insertion, Addition oder Inversion von 1 bis 50 Aminosäuren und mit einer katalytischen Aktivität für die Hydrolyse von Glutamin zu Glutaminsäure und Ammoniak;
(F) ein Protein mit einer Aminosäuresequenz der Aminosäurenummern 1-669 der SEQ ID NO: 22 mit Substitution, Deletion, Insertion, Addition oder Inversion von 1 bis 50 Aminosäuren und mit einer katalytischen Aktivität für die Hydrolyse von Glutamin zu Glutaminsäure und Ammoniak.

14. DNA nach Anspruch 2, die für ein in den nachfolgenden Punkten (E) oder (F) definiertes Protein kodiert:
(E) ein Protein mit einer Aminosäuresequenz der Aminosäurenummern 1-670 der SEQ ID NO: 2 mit Substitution, Deletion, Insertion, Addition oder Inversion von 1 bis 50 Aminosäuren und mit einer katalytischen Aktivität für die Hydrolyse von Glutamin zu Glutaminsäure und Ammoniak;
(F) ein Protein mit einer Aminosäuresequenz der Aminosäurenummern 1-669 der SEQ ID NO: 22 mit Substitution, Deletion, Insertion, Addition oder Inversion von 1 bis 50 Aminosäuren und mit einer katalytischen Aktivität für die Hydrolyse von Glutamin zu Glutaminsäure und Ammoniak.

15. Protein nach Anspruch 13, das durch die folgenden Punkte (G) oder (H) definiert ist:
(G) ein Protein mit einer Aminosäuresequenz der Aminosäurenummern 1-670 der SEQ ID NO: 2 mit Substitution, Deletion, Insertion, Addition oder Inversion von 1 bis 10 Aminosäuren und mit einer katalytischen Aktivität für die Hydrolyse von Glutamin zu Glutaminsäure und Ammoniak;
(H) ein Protein mit einer Aminosäuresequenz der Aminosäurenummern 1-669 der SEQ ID NO: 22 mit Substitution, Deletion, Insertion, Addition oder Inversion von 1 bis 10 Aminosäuren und mit einer katalytischen Aktivität für die Hydrolyse von Glutamin zu Glutaminsäure und Ammoniak.

16. DNA nach Anspruch 14, die für ein durch die folgenden Punkte (E) bis (F) definiertes Protein kodiert:
(E) ein Protein mit einer Aminosäuresequenz der Aminosäurenummern 1-670 der SEQ ID NO: 2 mit Substitution, Deletion, Insertion, Addition oder Inversion von 1 bis 10 Aminosäuren und mit einer katalytischen Aktivität für die Hydrolyse von Glutamin zu Glutaminsäure und Ammoniak;
(F) ein Protein mit einer Aminosäuresequenz der Aminosäurenummern 1-669 der SEQ ID NO: 22 mit Substitution, Deletion, Insertion, Addition oder Inversion von 1 bis 10 Aminosäuren und mit einer katalytischen Aktivität für die Hydrolyse von Glutamin zu Glutaminsäure und Ammoniak.

## Revendications

1. Protéine définie dans l'un quelconque des (A) à (D) ci-dessous:
(A) une protéine ayant une séquence d'aminoacides représentée par les numéros d'aminoacides 1-670 de SEQ ID N° 2;
(B) une protéine ayant une séquence d'aminoacides représentée par les numéros d'aminoacides 1-669 de SEQ ID N° 22;
(C) une protéine ayant une séquence d'aminoacides représentée par les numéros d'aminoacides 1-670 de SEQ ID N° 2 avec une substitution, une délétion, une insertion, une addition ou une inversion de 1 à 170 aminoacides, et ayant une activité de catalyse de l'hydrolyse de la glutamine en acide glutamique et en ammoniac;
(D) une protéine ayant une séquence d'aminoacides représentée par les numéros d'aminoacides 1-669 de SEQ ID N° 22 avec une substitution, une délétion, une insertion, une addition ou une inversion de 1 à 170 aminoacides, et ayant une activité de catalyse de l'hydrolyse de la glutamine en acide glutamique et en ammoniac.

2. ADN qui code pour une protéine définie dans l'un quelconque des (A) à (D) ci-dessous:
(A) une protéine ayant une séquence d'aminoacides représentée par les numéros d'aminoacides 1-670 de SEQ ID N° 2;
(B) une protéine ayant une séquence d'aminoacides représentée par les numéros d'aminoacides 1-669 de SEQ ID N° 22;
(C) une protéine ayant une séquence d'aminoacides représentée par les numéros d'aminoacides 1-670 de SEQ ID N° 2 avec une substitution, une délétion, une insertion, une addition ou une inversion de 1 à 170 aminoacides, et ayant une activité de catalyse de l'hydrolyse de la glutamine en acide glutamique et en ammoniac;
(D) une protéine ayant une séquence d'aminoacides représentée par les numéros d'aminoacides 1-669 de SEQ ID N° 22 avec une substitution, une délétion, une insertion, une addition ou une inversion de 1 à 170 aminoacides, et ayant une activité de catalyse de l'hydrolyse de la glutamine en acide glutamique et en ammoniac.

3. ADN selon la revendication 2, qui est un ADN défini dans l'un quelconque des (a) à (d) ci-dessous:
(a) un ADN qui contient des séquences de nucléotides représentées par les numéros de nucléotides 1174-1370, 1446-1741, 1800-2242, 2297-2880, 2932-3134, 3181-3324, 3380-3515, 3562-3628 de la séquence de nucléotides de SEQ ID N° 1 dans cet ordre;
(b) un ADN qui contient les séquences de nucléotides représentées par les numéros de nucléotides 1807-2000, 2061-2353, 2412-2854, 2915-3498, 3554-3756, 3806-3949, 3996-4131, 4180-4246 de la séquence de nucléotides de SEQ ID N° 21 dans cet ordre;
(c) un ADN qui s'hybride avec l'ADN de (a) dans des conditions stringentes, et qui code pour une protéine ayant une activité de catalyse de l'hydrolyse de glutamine en acide glutamique et en ammoniac;
(d) un ADN qui s'hybride avec l'ADN de (b) dans des conditions stringentes, et qui code pour une protéine ayant une activité de catalyse de l'hydrolyse de la glutamine en acide glutamique et en ammoniac, les conditions stringentes comprenant un lavage dans SSC × 1 et 0,1 % de SDS.

4. ADN selon la revendication 2 ou 3, qui a la séquence de nucléotides représentée par SEQ ID N° 1 ou SEQ ID N° 17.

5. ADN selon la revendication 2 ou 3, qui a une séquence de nucléotides représentée par SEQ ID N° 21 ou SEQ ID N° 25.

6. Vecteur recombiné contenant un ADN selon l'une quelconque des revendications 2 à 5.

7. Micro-organisme transformé dans lequel est introduit l'ADN selon l'une quelconque des revendications 2 à 5 ou un vecteur recombiné selon la revendication 6.

8. Micro-organisme transformé selon la revendication 7, qui est capable de produire une glutaminase.

9. Micro-organisme transformé selon la revendication 7 ou 8, qui est dérivé d'un champignon filamenteux ou d'une bactérie appartenant au genre *Escherichia.*

10. Procédé de production de glutaminase, qui comprend la culture du micro-organisme selon l'une quelconque des revendications 7 à 9 dans un milieu de culture pour produire de la glutaminase dans la culture.

11. Utilisation d'une protéine selon la revendication 1 pour le traitement d'aliments.

12. Utilisation d'un micro-organisme selon l'une quelconque des revendications 7 à 9 pour la production d'une sauce au soja, de miso, ou d'autres assaisonnements contenant des produits d'hydrolyse de protéines.

13. Protéine selon la revendication 1, qui est définie dans (E) ou (F) ci-dessous:
(E) une protéine ayant une séquence d'aminoacides représentée par les numéros d'aminoacides 1-670 de SEQ ID N° 2 avec une substitution, une délétion, une insertion, une addition ou une inversion de 1 à 50 aminoacides, et ayant une activité de catalyse de l'hydrolyse de la glutamine en acide glutamique et en ammoniac;
(F) une protéine ayant une séquence d'aminoacides représentée par les numéros d'aminoacides 1-669 de SEQ ID N° 22 avec une substitution, une délétion, une insertion, une addition ou une inversion de 1 à 50 aminoacides, et ayant une activité de catalyse de l'hydrolyse de la glutamine en acide glutamique et en ammoniac.

14. ADN selon la revendication 2, qui code pour une protéine définie dans (E) ou (F) ci-dessous:
(E) une protéine ayant une séquence d'aminoacides représentée par les numéros d'aminoacides 1-670 de SEQ ID N° 2 avec une substitution, une délétion, une insertion, une addition ou une inversion de 1 à 50 aminoacides, et ayant une activité de catalyse de l'hydrolyse de la glutamine en acide glutamique et en ammoniac;
(F) une protéine ayant une séquence d'aminoacides représentée par les numéros d'aminoacides 1-669 de SEQ ID N° 22 avec une substitution, une délétion, une insertion, une addition ou une inversion de 1 à 50 aminoacides, et ayant une activité de catalyse de l'hydrolyse de la glutamine en acide glutamique et en ammoniac.

15. Protéine selon la revendication 13, qui est définie dans (G) ou (H) ci-dessous:
(G) une protéine ayant une séquence d'aminoacides représentée par les numéros d'aminoacides 1-670 de SEQ ID N° 2 avec une substitution, une délétion, une insertion, une addition ou une inversion de 1 à 10 aminoacides, et ayant une activité de catalyse de l'hydrolyse de la glutamine en acide glutamique et en ammoniac;
(H) une protéine ayant une séquence d'aminoacides représentée par les numéros d'aminoacides 1-669 de SEQ ID N° 22 avec une substitution, une délétion, une insertion, une addition ou une inversion de 1 à 10 aminoacides, et ayant une activité de catalyse de l'hydrolyse de la glutamine en acide glutamique et en ammoniac.

16. ADN selon la revendication 14, qui code pour une protéine définie dans (E) à (F) ci-dessous:
(E) une protéine ayant une séquence d'aminoacides représentée par les numéros d'aminoacides 1-670 de SEQ ID N° 2 avec une substitution, une délétion, une insertion, une addition ou une inversion de 1 à 10 aminoacides, et ayant une activité de catalyse de l'hydrolyse de la glutamine en acide glutamique et en ammoniac;
(F) une protéine ayant une séquence d'aminoacides représentée par les numéros d'aminoacides 1-669 de SEQ ID N° 22 avec une substitution, une délétion, une insertion, une addition ou une inversion de 1 à 10 aminoacides, et ayant une activité de catalyse de l'hydrolyse de la glutamine en acide glutamique et en ammoniac.


### SEQUENCE LISTING

<110> Ajinomoto Co., Inc.
<120> A Novel Glutaminase, its Gene and
   a Method of Producing It
<130> EPA-53489
<141> 1999-05-12
<150> JP 10-134080
   <151> 1998-05-15
<150> JP 10-258974
   <151> 1998-09-11
<150> JP 10-292443
   <151> 1998-10-14
<150> JP 11-89157
   <151> 1999-03-30
<160> 26
   <170> PatentIn Ver. 2.0
<210> 1
   <211> 4013
   <212> DNA
   <213> Aspergillus oryzae
<220>
   <221> CDS
   <222> join(1174..1370,1446..1741, 1800..2242, 2297..2880, 2932..3134, 31 81..3324, 3380..3515, 3562..3628)
<220>
   <221> intron
   <222> (1371)..(1445)
<220>
   <221> intron
   <222> (1742)..(1799)
<220>
   <221> intron
   <222> (2243)..(2296)
<220>
   <221> intron
   <222> (2881)..(2931)
<220>
   <221> intron
   <222> (3135)..(3180)
<220>
   <221> intron
   <222> (3325)..(3379)
<220>
   <221> intron
   <222> (3516)..(3561)
f<220>
   <221> sig_peptide
   <222> (1174)..(1233)
<220>
   <221> mat_peptide
   <222> (1234..1370, 1446..1741, 1800..2242, 2297..2880, 2932..3134, 3181.. 3324, 3380..3515, 3562..3628)
<400> 1
   ctcgagagac tccttgccac ctgatactat cacaattgtc ggtcacacgc gactggctac 60
   tactacttcg taggcaccgt agtatacccc agtctcttta ggatggcaat accttgttgt 120
   atagatgttg tagtcatgta ggattccctg gagattccct gatcggacgg caagagtgac 180
   cccacttcca agtattgact ccatctcagc tcgatgtatc actttcctgt ttagggcacg 240
   ggagcatcac tgacggctct ctgcctcagg ccatgattgt ttcttttgtc aattcgactt 300
   ctccaaatcg agctgcagat ctgtcgaccc catcccagtt gatgcagtag ctcggctacc 360
   ggaagagatt tattcttagt cccttgttgg gattgggatt caccctcgct tctgtttctc 420
   accgtattta tatcgcgcaa tgagattgat ccggatataa aatgtctgtg atgcactctt 480
   tctcacgcac cgaggtaatc aatatcatat gctttcccct catatcactg ccgaaaagac 540
   taactcggtc taccccatag tcaccagcca ctagcgcttc ttgggcctct ccttgtttgc 600
   tcagtggatc taaagccaag actatcatgg ttagtgtcgg gttgtcttca ttagatcgtc 660
   tgcagcccca gagtgtatcg gcttaggact ggtcgagccc gacgcggcta aggataaggt 720
   acatactccc actctatcga cccttgcttg ttaatctccg atcttgtctc ctgtccaatt 780
   gtcgggcttc tcctggaatt ccaggtttct ttcacctgtc gggcagccgg atcgaggccg 840
   catgaattgc tcccccacag agactgacag gtcaggcgat attgggggag tcacaatcat 900
   gcgcgccccc attccgcatt ccgtttctcg accctcatgc agcgtgctaa acttccatag 960
   tccctcctga attgtctgcc ctgccctccg gtatgcgggc tggaccaact atataagtgt 1020
   gcctaacatt ccttcagcat tcttcaggcc cacattctcg gggcacgttt ttttggcgga 1080
   tctcgatcct actctttcat tctttgaaga aacctggaat tattacgtgt ataaatgaag 1140
   gatgtaccct gtgtgaatcc ctcaatacgg atc atg atg cat ttc ctc tcg ttc  1194
                                        Met Met His Phe Leu Ser Phe
                                        -20                 -15
   tgt ctg tcg gtg gcc tcc ctg gtg tct tac gcg gga gct gcg tca aca  1242
   Cys Leu Ser Val Ala Ser Leu Val Ser Tyr Ala Gly Ala Ala Ser Thr
               -10                 -5                   1
   ttc tcc cct gcg agg cca ccc gcc ctg tcc ttg gct gtc aaa tcg ccg  1290
   Phe Ser Pro Ala Arg Pro Pro Ala Leu Pro Leu Ala Val Lys Ser Pro
           5                  10                  15
   tac ttg agc aca tgg ctc tct gcg ggc aca gat ggc ggt aat gga ggg  1338
   Tyr Leu Ser Thr Trp Leu Ser Ala Gly Thr Asp Gly Gly Asn Gly Gly
    20                  25                  30                  35
   tac ctg gcc ggc caa tgg cct acc ttc tgg tt  gtgagtagtc           1380
   Tyr Leu Ala Gly Gln Trp Pro Thr Phe Trp Phe
                   40                  45
   ccgagctgta gaaatgaaga catccatctt gatgtacatt ggctaaacca cgtccctcgt 1440
   ggcag c ggc cag gtg acc ggc tgg gcg ggt cag atc cgg gtc gat aat  1488
           Gly Gln Val Thr Gly Trp Ala Gly Gln Ile Arg Val Asp Asn
                       50                  55                  60
   tcg acc tac aca tgg atg ggg gcg atc cct aac acc cct acg gtg aac  1536
   Ser Thr Tyr Thr Trp Met Gly Ala Ile Pro Asn Thr Pro Thr Val Asn
                   65                  70                  75
   cag aca tcc ttc gag tac acc tcg acg tcg agc gtg ttc acg atg cgt  1584
   Gln Thr Ser Phe Glu Tyr Thr Ser Thr Ser Ser Val Phe Thr Met Arg
               80                  85                  90
   gtt ggg gat atg gtg gaa atg aaa gtg aaa ttc ctg tcc cct atc aca  1632
   Val Gly Asp Met Val Glu Met Lys Val Lys Phe Leu Ser Pro Ile Thr
           95                 100                 105
   cca gat gat ctc cgg aga cag tcg ctt gtg ttt tcc tat ctg gac gta  1680
   Pro Asp Asp Leu Arg Arg Gln Ser Leu Val Phe Ser Tyr Leu Asp Val
       110                 115                 120
   gat gtc gaa tcg atc gac ggc aaa gcg cat gac ata cag gtg tac gca  1728
   Asp Val Glu Ser Ile Asp Gly Lys Ala His Asp Ile Gln Val Tyr Ala
   125                 130                 135                 140
   gac att tca gca g gtaagcaaga cgacaaccca cctggaacag tgcaaatatc    1781
   Asp Ile Ser Ala
   catctaaccg ggtcttag aa tgg gcg tcc ggg gac cga aac gcc att gcg   1831
                          Glu Trp Ala Ser Gly Asp Arg Asn Ala Ile Ala
                          145                 150                 155
   cag tgg gac tat ggt gtc aca gat gat ggc gtt gcc tat cac aag gtt  1879
   Gln Trp Asp Tyr Gly Val Thr Asp Asp Gly Val Ala Tyr His Lys Val
                   160                 165                 170
   tac cgc caa acg cag ctg ctg ttt tcc gaa aac act gag cag gcc gaa  1927
   Tyr Arg Gln Thr Gln Leu Leu Phe Ser Glu Asn Thr Glu Gln Ala Glu
               175                 180                 185
   tgg ggc gag tgg tac tgg gcc aca gac gac caa gat ggt ctg agc tac  1975
   Trp Gly Glu Trp Tyr Trp Ala Thr Asp Asp Gln Asp Gly Leu Ser Tyr
           190                 195                 200
   cag tcc gga ccg gat gtt gat gtg cga ggg gca ttc gca aag aac gga  2023
   Gln Ser Gly Pro Asp Val Asp Val Arg Gly Ala Phe Ala Lys Asn Gly
       205                 210                 215
   aag ttg gcg aat tcg gat gat aaa aat tat cgt gca atc tcg acc aat  2071
   Lys Leu Ala Asn Ser Asp Asp Lys Asn Tyr Arg Ala Ile Ser Thr Asn
   220                 225                 230                 235
   tgg ccc gtg ttt gcc ttc tcc cgc gat ctt ggc tcg gtg aag acg tct  2119
   Trp Pro Val Phe Ala Phe Ser Arg Asp Leu Gly Ser Val Lys Thr Ser
                   240                 245                 250
   gct ggc acg tta ttc tcc att ggc ctt gcg cag gac agt gcc ata cag  2167
   Ala Gly Thr Leu Phe Ser Ile Gly Leu Ala Gln Asp Ser Ala Ile Gln
               255                 260                 265
   tac agt ggg aaa cct gaa ggg aca act gtg atg cct tca ctc tgg aag  2215
   Tyr Ser Gly Lys Pro Glu Gly Thr Thr Val Met Pro Ser Leu Trp Lys
           270                 275                 280
   agc tac ttc agc act gcg act gct gcg gtaagtggcc cactgctgtt        2262
   Ser Tyr Phe Ser Thr Ala Thr Ala Ala
       285                 290
   tcggacctag aacataatct gaccatctat gtag ctt gag ttc ttc cat cat    2314
                                         Leu Glu Phe Phe His His
                                                     295
   gat tat gct gct gca gct gca cta tcg aag gat ctc gat gac cgg ata  2362
   Asp Tyr Ala Ala Ala Ala Ala Leu Ser Lys Asp Leu Asp Asp Arg Ile
       300                 305                 310
   tcc aag gat tcc att gat gcc gct ggc cag gac tac ctg aca atc acc  2410
   Ser Lys Asp Ser Ile Asp Ala Ala Gly Gln Asp Tyr Leu Thr Ile Thr
   315                 320                 325                 330
   tcc ctc acg gtc cgt caa gtc ttt gct gcc gtg caa ttg acc ggc acg  2458
   Ser Leu Thr Val Arg Gln Val Phe Ala Ala Val Gln Leu Thr Gly Thr
                   335                 340                 345
   ccc gag gac ccc tac atc ttc atg aag gag atc tcg tcc aat ggc aac     2506
   Pro Glu Asp Pro Tyr Ile Phe Met Lys Glu Ile Ser Ser Asn Gly Asn
               350                 355                 360
   atg aac act gtg gac gtc atc ttc ccc gct cac ccg atc ttt ttg tac     2554
   Met Asn Thr Val Asp Val Ile Phe Pro Ala His Pro Ile Phe Leu Tyr
           365                 370                 375
   acc aat ccc gag ctc ctc aaa ctg att ctg aag cca atc tat gag att     2602
   Thr Asn Pro Glu Leu Leu Lys Leu Ile Leu Lys Pro Ile Tyr Glu Ile
       380                 385                 390
   caa gag aac gga aag tat ccc aac aca tac gcc atg cac gat att gga     2650
   Gln Glu Asn Gly Lys Tyr Pro Asn Thr Tyr Ala Met His Asp Ile Gly
   395                 400                 405                 410
   acc cac tac ccg aac gcc acg ggc cat cct aag ggc gac gac gag aaa     2698
   Thr His Tyr Pro Asn Ala Thr Gly His Pro Lys Gly Asp Asp Glu Lys
                   415                 420                 425
   atg cca ctc gag gag tgt gga aac atg gtt atc atg gcc ctt gcc tac     2746
   Met Pro Leu Glu Glu Cys Gly Asn Met Val Ile Met Ala Leu Ala Tyr
               430                 435                 440
   gcc cag aag gcc aag gac aac gac tat ctt tca cag cac tat ccc atc     2794
   Ala Gln Lys Ala Lys Asp Asn Asp Tyr Leu Ser Gln His Tyr Pro Ile
           445                 450                 455
   ctc aac aaa tgg aca aca tac ctc gtc gag gat tct att tac ccg gcg     2842
   Leu Asn Lys Trp Thr Thr Tyr Leu Val Glu Asp Ser Ile Tyr Pro Ala
       460                 465                 470
   aac cag atc tct acg gat gac ttt gct ggc tcg cta gc  gtaagtgata     2890
   Asn Gln Ile Ser Thr Asp Asp Phe Ala Gly Ser Leu Ala
   475                 480                 485
   tacatacacg acacaggcgg tgatactaat agtatgtaca g a aac cag acc aac     2944
                                                   Asn Gln Thr Asn
                                                               490
   ctg gca ttg aag gga atc att gga atc cag gca atg gct gtg atc agc     2992
   Leu Ala Leu Lys Gly Ile Ile Gly Ile Gln Ala Met Ala Val Ile Ser
               495                 500                 505
   aat acg aca gga cac ccg gac gat gcc tcc aac cac tcc agc att gcc     3040
   Asn Thr Thr Gly His Pro Asp Asp Ala Ser Asn His Ser Ser Ile Ala
           510                 515                 520
   aag gac tac atc gcg agg tgg cag aca cta ggc gta gct cac gat gcc     3088
   Lys Asp Tyr Ile Ala Arg Trp Gln Thr Leu Gly Val Ala His Asp Ala
       525                 530                 535
   aat cct ccg cat aca acg ctg tcg tac gga gcg aac gag act cat g      3134
   Asn Pro Pro His Thr Thr Leu Ser Tyr Gly Ala Asn Glu Thr His
   540                545                550
   gtcagttagc cgctccgggt gcacttataa tactgacttt ctccag  gg ctt ctg    3188
                                                      Gly Leu Leu
                                                      555
   tac aat ctg tat gcg gat cgt gaa ttg ggc ttg aac ttg gtt cct cag   3236
   Tyr Asn Leu Tyr Ala Asp Arg Glu Leu Gly Leu Asn Leu Val Pro Gln
           560                 565                 570
   tcg gtc tat gac atg caa aac acc ttc tat ccg acg gtg aag gag aag   3284
   Ser Val Tyr Asp Met Gln Asn Thr Phe Tyr Pro Thr Val Lys Glu Lys
       575                 580                 585
   tat gga gtg ccg ctc gat act cga cac gtg tac act aag g             3324
   Tyr Gly Val Pro Leu Asp Thr Arg His Val Tyr Thr Lys
   590                 595                 600
   gtaagctcga tatgttcttt ctaatgtttg acattgaata ttgacttgtc cccag  cg  3381
                                                                 Ala
   gat tgg gag ctt ttc aca gct gcg gtt gcg tcg gag agt gtc cga gac   3429
   Asp Trp Glu Leu Phe Thr Ala Ala Val Ala Ser Glu Ser Val Arg Asp
       605                 610                 615
   atg ttc cac cag gcg ctc gcg acg tgg atc aac gag aca ccg acc aac   3477
   Met Phe His Gln Ala Leu Ala Thr Trp Ile Asn Glu Thr Pro Thr Asn
   620                 625                 630                 635
   cgt gcc ttt acg gat ctc tat gat acc caa act gga aa  gtaagtgttt    3525
   Arg Ala Phe Thr Asp Leu Tyr Asp Thr Gln Thr Gly Asn
                   640                 645
   gccaaggggc tgcttgggcc ttgctgacca atatag t tat ccg gcg ggc att     3577
                                             Tyr Pro Ala Gly Ile
                                                 650
   acg ttc att gcg cgg ccc gtc atg ggt ggt gcc ttt gcg ttg tta att   3625
   Thr Phe Ile Ala Arg Pro Val Met Gly Gly Ala Phe Ala Leu Leu Ile
       655                 660                 665
   ctc tagagtcgtt tcattgtata ttgattttat tcgcttctgg gcgcgagtgg        3678
   Leu
   670
   agacacttgc ttactttgtt tccaatttta ttattaccgt ggctatggga ccagattgac 3738
   cgttgttaat agcgtacctc atacatagca tttttattct gcaaatagtg ttgttgtatt 3798
   tgggtctcca ataataatgc gttcgtagac gatgctccaa aggaactatc tggtctgcaa 3858
   gctgcttata tcaagcatat ataaagatct acgtatccag tcgtgcttat ccaagtggct 3918
   ctggccatct accgcagatc gtaagtggac tcgaccgagc catcccacgc atcaaaagcg 3978
   cgaataagat ctacaaagtc agcaatcata ctcct                            4013
<210> 2
   <211> 690
   <212> PRT
<213> Aspergillus oryzae
<400> 2
   Met Met His Phe Leu Ser Phe Cys Leu Ser Val Ala Ser Leu Val Ser
   -20                 -15                 -10                  -5
   Tyr Ala Gly Ala Ala Ser Thr Phe Ser Pro Ala Arg Pro Pro Ala Leu
                     1               5                  10
   Pro Leu Ala Val Lys Ser Pro Tyr Leu Ser Thr Trp Leu Ser Ala Gly
           15                  20                  25
   Thr Asp Gly Gly Asn Gly Gly Tyr Leu Ala Gly Gln Trp Pro Thr Phe
       30                  35                  40
   Trp Phe Gly Gln Val Thr Gly Trp Ala Gly Gln Ile Arg Val Asp Asn
   45                  50                  55                  60
   Ser Thr Tyr Thr Trp Met Gly Ala Ile Pro Asn Thr Pro Thr Val Asn
                   65                  70                  75
   Gln Thr Ser Phe Glu Tyr Thr Ser Thr Ser Ser Val Phe Thr Met Arg
               80                  85                  90
   Val Gly Asp Met Val Glu Met Lys Val Lys Phe Leu Ser Pro Ile Thr
           95                  100                 105
   Pro Asp Asp Leu Arg Arg Gln Ser Leu Val Phe Ser Tyr Leu Asp Val
       110                 115                 120
   Asp Val Glu Ser Ile Asp Gly Lys Ala His Asp Ile Gln Val Tyr Ala
   125                 130                 135                 140
   Asp Ile Ser Ala Glu Trp Ala Ser Gly Asp Arg Asn Ala Ile Ala Gln
                   145                 150                 155
   Trp Asp Tyr Gly Val Thr Asp Asp Gly Val Ala Tyr His Lys Val Tyr
               160                 165                 170
   Arg Gln Thr Gln Leu Leu Phe Ser Glu Asn Thr Glu Gln Ala Glu Trp
           175                 180                 185
   Gly Glu Trp Tyr Trp Ala Thr Asp Asp Gln Asp Gly Leu Ser Tyr Gln
       190                 195                 200
   Ser Gly Pro Asp Val Asp Val Arg Gly Ala Phe Ala Lys Asn Gly Lys
   205                 210                 215                 220
   Leu Ala Asn Ser Asp Asp Lys Asn Tyr Arg Ala Ile Ser Thr Asn Trp
                   225                 230                 235
   Pro Val Phe Ala Phe Ser Arg Asp Leu Gly Ser Val Lys Thr Ser Ala
               240                 245                 250
   Gly Thr Leu Phe Ser Ile Gly Leu Ala Gln Asp Ser Ala Ile Gln Tyr
           255                 260                 265
   Ser Gly Lys Pro Glu Gly Thr Thr Val Met Pro Ser Leu Trp Lys Ser
       270                 275                 280
   Tyr Phe Ser Thr Ala Thr Ala Ala Leu Glu Phe Phe His His Asp Tyr
   285                 290                 295                 300
   Ala Ala Ala Ala Ala Leu Ser Lys Asp Leu Asp Asp Arg Ile Ser Lys
   305 310 315
   Asp Ser Ile Asp Ala Ala Gly Gln Asp Tyr Leu Thr Ile Thr Ser Leu
   320 325 330
   Thr Val Arg Gln Val Phe Ala Ala Val Gln Leu Thr Gly Thr Pro Glu
   335 340 345
   Asp Pro Tyr Ile Phe Met Lys Glu Ile Ser Ser Asn Gly Asn Met Asn
   350 355 360
   Thr Val Asp Val Ile Phe Pro Ala His Pro Ile Phe Leu Tyr Thr Asn
   365 370 375 380
   Pro Glu Leu Leu Lys Leu Ile Leu Lys Pro Ile Tyr Glu Ile Gln Glu
   385 390 395
   Asn Gly Lys Tyr Pro Asn Thr Tyr Ala Met His Asp Ile Gly Thr His
   400 405 410
   Tyr Pro Asn Ala Thr Gly His Pro Lys Gly Asp Asp Glu Lys Met Pro
   415 420 425
   Leu Glu Glu Cys Gly Asn Met Val Ile Met Ala Leu Ala Tyr Ala Gln
   430 435 440
   Lys Ala Lys Asp Asn Asp Tyr Leu Ser Gln His Tyr Pro Ile Leu Asn
   445 450 455 460
   Lys Trp Thr Thr Tyr Leu Val Glu Asp Ser Ile Tyr Pro Ala Asn Gln
   465 470 475
   Ile Ser Thr Asp Asp Phe Ala Gly Ser Leu Ala Asn Gln Thr Asn Leu
   480 485 490
   Ala Leu Lys Gly Ile Ile Gly Ile Gln Ala Met Ala Val Ile Ser Asn
   495 500 505
   Thr Thr Gly His Pro Asp Asp Ala Ser Asn His Ser Ser Ile Ala Lys
   510 515 520
   Asp Tyr Ile Ala Arg Trp Gln Thr Leu Gly Val Ala His Asp Ala Asn
   525 530 535 540
   Pro Pro His Thr Thr Leu Ser Tyr Gly Ala Asn Glu Thr His Gly Leu
   545 550 555
   Leu Tyr Asn Leu Tyr Ala Asp Arg Glu Leu Gly Leu Asn Leu Val Pro
   560 565 570
   Gln Ser Val Tyr Asp Met Gln Asn Thr Phe Tyr Pro Thr Val Lys Glu
   575 580 585
   Lys Tyr Gly Val Pro Leu Asp Thr Arg His Val Tyr Thr Lys Ala Asp
   590 595 600
   Trp Glu Leu Phe Thr Ala Ala Val Ala Ser Glu Ser Val Arg Asp Met
   605 610 615 620
   Phe His Gln Ala Leu Ala Thr Trp Ile Asn Glu Thr Pro Thr Asn Arg
   625 630 635
   Ala Phe Thr Asp Leu Tyr Asp Thr Gln Thr Gly Asn Tyr Pro Ala Gly
   640 645 650
   Ile Thr Phe Ile Ala Arg Pro Val Met Gly Gly Ala Phe Ala Leu Leu
   655 660 665
   Ile Leu
   670
<210> 3
   <211> 17
   <212> PRT
   <213> Aspergillus oryzae
<400> 3
   Ala Ser Thr Phe Ser Pro Ala Arg Pro Pro Ala Leu Pro Leu Ala Val
   1 5 10 15
   Lys
<210> 4
   <211> 10
   <212> PRT
   <213> Aspergillus oryzae
<220>
   <221> misc_feature
   <222> (2)
   <223> Xaa=Gly or Pro
<220>
   <221> misc_feature
   <222> (3)
   <223> Xaa=Asn or Val
<220>
   <221> misc_feature
   <222> (4)
   <223> Xaa=Thr or Pro
<220>
   <221> misc_feature
   <222> (8)
   <223> Xaa=Arg or Ser
<400> 4
   Tyr Xaa Xaa Xaa Tyr Ala Met Xaa Asp Ile
   1 5 10
<210> 5
   <211> 9
   <212> PRT
   <213> Aspergillus oryzae
<220>
   <221> misc_feature
   <222> (4)
   <223> Xaa=Thr or Gly
<220>
   <221> misc_feature
   <222> (8)
   <223> Xaa is uncertain
<400> 5
   Val Gln Tyr Xaa Glu Tyr Asp Xaa Tyr
   1 5
<210> 6
   <211> 14
   <212> PRT
   <213> Aspergillus oryzae
<400> 6
   Asp Asn Asp Tyr Leu Ser Gln His Tyr Pro Ile Leu Asn Lys
   1 5 10
<210> 7
   <211> 15
   <212> PRT
   <213> Aspergillus oryzae
<400> 7
   Trp Thr Ala Tyr Leu Val Glu Asp Thr Ile Tyr Pro Ala Asn Gln
   1 5 10 15
<210> 8
   <211> 10
   <212> PRT
   <213> Aspergillus oryzae
<400> 8
   Val Leu Leu Gln Ser Ala Ile Glu Gly His
   1 5 10
<210> 9
   <211> 10
   <212> PRT
   <213> Aspergillus oryzae
<400> 9
   Gly Ile Ile Gly Ile Gln Ala Met Ala Val
   1               5                   10
<210> 10
   <211> 10
   <212> PRT
   <213> Aspergillus oryzae
<220>
   <221> misc_feature
   <222> (1,6,8,9)
   <223> Xaa is uncertain
<400> 10
   Xaa Ile Leu Lys Phe Xaa Tyr Xaa Xaa Gln
   1               5                   10
<210> 11
   <211> 30
   <212> DNA
   <213> Artificial Sequence
<220>
   <223> Description of Artificial Sequence:primer
<400> 11
   taccccaaca cctatgctat gcgcgatatc 30
<210> 12
   <211> 36
   <212> DNA
   <213> Artificial Sequence
<220>
   <223> Description of Artificial Sequence:primer
<400> 12
   ttggttcgcc ggataaatag tatcttcgac caagta 36
<210> 13
   <211> 29
   <212> DNA
   <213> Artificial Sequence
<220>
   <223> Description of Artificial Sequence:primer
<400> 13
   gatcatgatg catttcctct cgttctgtc 29
<210> 14
   <211> 28
   <212> DNA
   <213> Artificial Sequence
<220>
   <223> Description of Artificial Sequence:primer
<400> 14
   gcaaagtcat ccgtagagat ctggttcg 28
<210> 15
   <211> 30
   <212> DNA
   <213> Artificial Sequence
<220>
   <223> Description of Artificial Sequence:primer
<400> 15
   ggcgaaccag atctctacgg atgactttgc 30
<210> 16
   <211> 21
   <212> DNA
<213> Artificial Sequence
<220>
   <223> Description of Artificial Sequence:primer
<400> 16
   ctgatctaga cctgcaggct c 21
<210> 17
   <211> 2269
   <212> DNA
   <213> Aspergillus oryzae
<220>
   <221> CDS
   <222> (1)..(2070)
<220>
   <221> sig_peptide
   <222> (1)..(60)
<220>
   <221> mat_peptide
   <222> (61)..(2070)
<400> 17
   atg atg cat ttc ctc tcg ttc tgt ctg tcg gtg gcc tcc ctg gtg tct    48
   Met Met His Phe Leu Ser Phe Cys Leu Ser Val Ala Ser Leu Val Ser
   -20                 -15                 -10                  -5
   tac gcc gga gct gcg tca aca ttc tcc cct gcg agg cca ccc gcc ctg    96
   Tyr Ala Gly Ala Ala Ser Thr Phe Ser Pro Ala Arg Pro Pro Ala Leu
                -1   1                  5                  10
   ccc ttg gct gtc aaa tcg ccg tac ttg agc aca tgg ctc tct gcg ggc   144
   Pro Leu Ala Val Lys Ser Pro Tyr Leu Ser Thr Trp Leu Ser Ala Gly
            15                  20                  25
   aca gat ggc ggt aat gga ggg tac ctg gcc ggc caa tgg cct acc ttc   192
   Thr Asp Gly Gly Asn Gly Gly Tyr Leu Ala Gly Gln Trp Pro Thr Phe
        30                  35                  40
   tgg ttc ggc cag gtg acc ggc tgg gcg ggt cag atc cgg gtc gat aat   240
   Trp Phe Gly Gln Val Thr Gly Trp Ala Gly Gln Ile Arg Val Asp Asn
    45                  50                  55                  60
   tcg acc tac aca tgg atg ggg gcg atc cct aac acc cct acg gtg aac   288
   Ser Thr Tyr Thr Trp Met Gly Ala Ile Pro Asn Thr Pro Thr Val Asn
                   65                  70                  75
   cag aca tcc ttc gag tac acc tcg acg tcg agc gtg ttc acg atg cgt    336
   Gln Thr Ser Phe Glu Tyr Thr Ser Thr Ser Ser Val Phe Thr Met Arg
               80                  85                  90
   gtt ggg gat atg gtg gaa atg aaa gtg aaa ttc ctg tcc cct atc aca    384
   Val Gly Asp Met Val Glu Met Lys Val Lys Phe Leu Ser Pro Ile Thr
           95                  100                 105
   cca gat gat ctc cgg aga cag tcg ctt gtg ttt tcc tat ctg gac gta    432
   Pro Asp Asp Leu Arg Arg Gln Ser Leu Val Phe Ser Tyr Leu Asp Val
       110                 115                 120
   gat gtc gaa tcg atc gac ggc aaa gcg cat gac ata cag gtg tac gca    480
   Asp Val Glu Ser Ile Asp Gly Lys Ala His Asp Ile Gln Val Tyr Ala
   125                 130                 135                 140
   gac att tca gca gaa tgg gcg tcc ggg gac cga aac gcc att gcg cag    528
   Asp Ile Ser Ala Glu Trp Ala Ser Gly Asp Arg Asn Ala Ile Ala Gln
                   145                 150                 155
   tgg gac tat ggt gtc aca gat gat ggc gtt gcc tat cac aag gtt tac    576
   Trp Asp Tyr Gly Val Thr Asp Asp Gly Val Ala Tyr His Lys Val Tyr
               160                 165                 170
   cgc caa acg cag ctg ctg ttt tcc gaa aac act gag cag gcc gaa tgg    624
   Arg Gln Thr Gln Leu Leu Phe Ser Glu Asn Thr Glu Gln Ala Glu Trp
           175                 180                 185
   ggc gag tgg tac tgg gcc aca gac gac caa gat ggt ctg agc tac cag    672
   Gly Glu Trp Tyr Trp Ala Thr Asp Asp Gln Asp Gly Leu Ser Tyr Gln
       190                 195                 200
   tcc gga ccg gat gtt gat gtg cga ggg gca ttc gca aag aac gga aag    720
   Ser Gly Pro Asp Val Asp Val Arg Gly Ala Phe Ala Lys Asn Gly Lys
   205                 210                 215                 220
   ttg gcg aat tcg gat gat aaa aat tat cgt gca atc tcg acc aat tgg    768
   Leu Ala Asn Ser Asp Asp Lys Asn Tyr Arg Ala Ile Ser Thr Asn Trp
                   225                 230                 235
   ccc gtg ttt gcc ttc tcc cgc gat ctt ggc tcg gtg aag acg tct gct    816
   Pro Val Phe Ala Phe Ser Arg Asp Leu Gly Ser Val Lys Thr Ser Ala
               240                 245                 250
   ggc acg tta ttc tcc att ggc ctt gcg cag gac agt gcc ata cag tac    864
   Gly Thr Leu Phe Ser Ile Gly Leu Ala Gln Asp Ser Ala Ile Gln Tyr
           255                 260                 265
   agt ggg aaa cct gaa ggg aca act gtg atg cct tca ctc tgg aag agc    912
   Ser Gly Lys Pro Glu Gly Thr Thr Val Met Pro Ser Leu Trp Lys Ser
       270                 275                 280
   tac ttc agc act gcg act gct gcg ctt gag ttc ttc cat cat gat tat    960
   Tyr Phe Ser Thr Ala Thr Ala Ala Leu Glu Phe Phe His His Asp Tyr
   285                 290                 295                 300
   gct gct gca gct gca cta tcg aag gat ctc gat gac cgg ata tcc aag     1008
   Ala Ala Ala Ala Ala Leu Ser Lys Asp Leu Asp Asp Arg Ile Ser Lys
                   305                 310                 315
   gat tcc att gat gcc gct ggc cag gac tac ctg aca atc acc tcc ctc     1056
   Asp Ser Ile Asp Ala Ala Gly Gln Asp Tyr Leu Thr Ile Thr Ser Leu
               320                 325                 330
   acg gtc cgt caa gtc ttt gct gcc gtg caa ttg acc ggc acg ccc gag     1104
   Thr Val Arg Gln Val Phe Ala Ala Val Gln Leu Thr Gly Thr Pro Glu
           335                 340                 345
   gac ccc tac atc ttc atg aag gag atc tcg tcc aat ggc aac atg aac     1152
   Asp Pro Tyr Ile Phe Met Lys Glu Ile Ser Ser Asn Gly Asn Met Asn
       350                 355                 360
   act gtg gac gtc atc ttc ccc gct cac ccg atc ttt ttg tac acc aat     1200
   Thr Val Asp Val Ile Phe Pro Ala His Pro Ile Phe Leu Tyr Thr Asn
   365                 370                 375                 380
   ccc gag ctc ctc aaa ctg att ctg aag cca atc tat gag att caa gag     1248
   Pro Glu Leu Leu Lys Leu Ile Leu Lys Pro Ile Tyr Glu Ile Gln Glu
                   385                 390                 395
   aac gga aag tat ccc aac aca tac gcc atg cac gat att gga acc cac     1296
   Asn Gly Lys Tyr Pro Asn Thr Tyr Ala Met His Asp Ile Gly Thr His
               400                 405                 410
   tac ccg aac gcc acg ggc cat cct aag ggc gac gac gag aaa atg cca     1344
   Tyr Pro Asn Ala Thr Gly His Pro Lys Gly Asp Asp Glu Lys Met Pro
           415                 420                 425
   ctc gag gag tgt gga aac atg gtt atc atg gcc ctt gcc tac gcc cag     1392
   Leu Glu Glu Cys Gly Asn Met Val Ile Met Ala Leu Ala Tyr Ala Gln
       430                 435                 440
   aag gcc aag gac aac gac tat ctt tca cag cac tat ccc atc ctc aac     1440
   Lys Ala Lys Asp Asn Asp Tyr Leu Ser Gln His Tyr Pro Ile Leu Asn
   445                 450                 455                 460
   aaa tgg aca aca tac ctc gtc gag gat tct att tac ccg gcg aac cag     1488
   Lys Trp Thr Thr Tyr Leu Val Glu Asp Ser Ile Tyr Pro Ala Asn Gln
                   465                 470                 475
   atc tct acg gat gac ttt gct ggc tcg cta gca aac cag acc aac ctg     1536
   Ile Ser Thr Asp Asp Phe Ala Gly Ser Leu Ala Asn Gln Thr Asn Leu
               480                 485                 490
   gca ttg aag gga atc att gga atc cag gca atg gct gtg atc agc aat     1584
   Ala Leu Lys Gly Ile Ile Gly Ile Gln Ala Met Ala Val Ile Ser Asn
           495                 500                 505
   acg aca gga cac ccg gac gat gcc tcc aac cac tcc agc att gcc aag     1632
   Thr Thr Gly His Pro Asp Asp Ala Ser Asn His Ser Ser Ile Ala Lys
       510                 515                 520
   gac tac atc gcg agg tgg cag aca cta ggc gta gct cac gat gcc aat     1680
   Asp Tyr Ile Ala Arg Trp Gln Thr Leu Gly Val Ala His Asp Ala Asn
   525                 530                 535                 540
   cct ccg cat aca acg ctg tcg tac gga gcg aac gag act cat ggg ctt     1728
   Pro Pro His Thr Thr Leu Ser Tyr Gly Ala Asn Glu Thr His Gly Leu
                   545                 550                 555
   ctg tac aat ctg tat gcg gat cgt gaa ttg ggc ttg aac ttg gtt cct     1776
   Leu Tyr Asn Leu Tyr Ala Asp Arg Glu Leu Gly Leu Asn Leu Val Pro
               560                 565                 570
   cag tcg gtc tat gac atg caa aac acc ttc tat ccg acg gtg aag gag     1824
   Gln Ser Val Tyr Asp Met Gln Asn Thr Phe Tyr Pro Thr Val Lys Glu
           575                 580                 585
   aag tat gga gtg ccg ctc gat act cga cac gtg tac act aag gcg gat     1872
   Lys Tyr Gly Val Pro Leu Asp Thr Arg His Val Tyr Thr Lys Ala Asp
       590                 595                 600
   tgg gag ctt ttc aca gct gcg gtt gcg tcg gag agt gtc cga gac atg     1920
   Trp Glu Leu Phe Thr Ala Ala Val Ala Ser Glu Ser Val Arg Asp Met
   605                 610                 615                 620
   ttc cac cag gcg ctc gcg acg tgg atc aac gag aca ccg acc aac cgt     1968
   Phe His Gln Ala Leu Ala Thr Trp Ile Asn Glu Thr Pro Thr Asn Arg
                   625                 630                 635
   gcc ttt acg gat ctc tat gat acc caa act gga aat tat ccg gcg ggc     2016
   Ala Phe Thr Asp Leu Tyr Asp Thr Gln Thr Gly Asn Tyr Pro Ala Gly
               640                 645                 650
   att acg ttc att gcg cgg ccc gtc atg ggt ggt gcc ttt gcg ttg tta     2064
   Ile Thr Phe Ile Ala Arg Pro Val Met Gly Gly Ala Phe Ala Leu Leu
           655                 660                 665
   att ctc tagagtcgtt tcattgtata ttgattttat tcgcttctgg gcgcgagtgg      2120
   Ile Leu
       670
   agacacttgc ttactttgtt tccaatttta ttattaccgt ggctatggga ccagattgac 2180
   cgttgttaat agcgtacctc atacatagca tttttattct gcaaaaaaaa aaaaaaaaaa  2240
   aaaaaaaaaa aaaaaaaaaa aaaaaaaaa                                   2269
<210> 18
   <211> 690
   <212> PRT
   <213> Aspergillus oryzae
<400> 18
   Met Met His Phe Leu Ser Phe Cys Leu Ser Val Ala Ser Leu Val Ser
   -20                 -15                 -10                  -5
   Tyr Ala Gly Ala Ala Ser Thr Phe Ser Pro Ala Arg Pro Pro Ala Leu
               -1   1               5                  10
   Pro Leu Ala Val Lys Ser Pro Tyr Leu Ser Thr Trp Leu Ser Ala Gly
   15 20 25
   Thr Asp Gly Gly Asn Gly Gly Tyr Leu Ala Gly Gln Trp Pro Thr Phe
   30 35 40
   Trp Phe Gly Gln Val Thr Gly Trp Ala Gly Gln Ile Arg Val Asp Asn
   45 50 55 60
   Ser Thr Tyr Thr Trp Met Gly Ala Ile Pro Asn Thr Pro Thr Val Asn
   65 70 75
   Gln Thr Ser Phe Glu Tyr Thr Ser Thr Ser Ser Val Phe Thr Met Arg
   80 85 90
   Val Gly Asp Met Val Glu Met Lys Val Lys Phe Leu Ser Pro Ile Thr
   95 100 105
   Pro Asp Asp Leu Arg Arg Gln Ser Leu Val Phe Ser Tyr Leu Asp Val
   110 115 120
   Asp Val Glu Ser Ile Asp Gly Lys Ala His Asp Ile Gln Val Tyr Ala
   125 130 135 140
   Asp Ile Ser Ala Glu Trp Ala Ser Gly Asp Arg Asn Ala Ile Ala Gln
   145 150 155
   Trp Asp Tyr Gly Val Thr Asp Asp Gly Val Ala Tyr His Lys Val Tyr
   160 165 170
   Arg Gln Thr Gln Leu Leu Phe Ser Glu Asn Thr Glu Gln Ala Glu Trp
   175 180 185
   Gly Glu Trp Tyr Trp Ala Thr Asp Asp Gln Asp Gly Leu Ser Tyr Gln
   190 195 200
   Ser Gly Pro Asp Val Asp Val Arg Gly Ala Phe Ala Lys Asn Gly Lys
   205 210 215 220
   Leu Ala Asn Ser Asp Asp Lys Asn Tyr Arg Ala Ile Ser Thr Asn Trp
   225 230 235
   Pro Val Phe Ala Phe Ser Arg Asp Leu Gly Ser Val Lys Thr Ser Ala
   240 245 250
   Gly Thr Leu Phe Ser Ile Gly Leu Ala Gln Asp Ser Ala Ile Gln Tyr
   255 260 265
   Ser Gly Lys Pro Glu Gly Thr Thr Val Met Pro Ser Leu Trp Lys Ser
   270 275 280
   Tyr Phe Ser Thr Ala Thr Ala Ala Leu Glu Phe Phe His His Asp Tyr
   285 290 295 300
   Ala Ala Ala Ala Ala Leu Ser Lys Asp Leu Asp Asp Arg Ile Ser Lys
   305 310 315
   Asp Ser Ile Asp Ala Ala Gly Gln Asp Tyr Leu Thr Ile Thr Ser Leu
   320 325 330
   Thr Val Arg Gln Val Phe Ala Ala Val Gln Leu Thr Gly Thr Pro Glu
   335 340 345
   Asp Pro Tyr Ile Phe Met Lys Glu Ile Ser Ser Asn Gly Asn Met Asn
       350                 355                 360
   Thr Val Asp Val Ile Phe Pro Ala His Pro Ile Phe Leu Tyr Thr Asn
   365                 370                 375                 380
   Pro Glu Leu Leu Lys Leu Ile Leu Lys Pro Ile Tyr Glu Ile Gln Glu
                   385                 390                 395
   Asn Gly Lys Tyr Pro Asn Thr Tyr Ala Met His Asp Ile Gly Thr His
               400                 405                 410
   Tyr Pro Asn Ala Thr Gly His Pro Lys Gly Asp Asp Glu Lys Met Pro
           415                 420                 425
   Leu Glu Glu Cys Gly Asn Met Val Ile Met Ala Leu Ala Tyr Ala Gln
       430                 435                 440
   Lys Ala Lys Asp Asn Asp Tyr Leu Ser Gln His Tyr Pro Ile Leu Asn
   445                 450                 455                 460
   Lys Trp Thr Thr Tyr Leu Val Glu Asp Ser Ile Tyr Pro Ala Asn Gln
                   465                 470                 475
   Ile Ser Thr Asp Asp Phe Ala Gly Ser Leu Ala Asn Gln Thr Asn Leu
               480                 485                 490
   Ala Leu Lys Gly Ile Ile Gly Ile Gln Ala Met Ala Val Ile Ser Asn
           495                 500                 505
   Thr Thr Gly His Pro Asp Asp Ala Ser Asn His Ser Ser Ile Ala Lys
       510                 515                 520
   Asp Tyr Ile Ala Arg Trp Gln Thr Leu Gly Val Ala His Asp Ala Asn
   525                 530                 535                 540
   Pro Pro His Thr Thr Leu Ser Tyr Gly Ala Asn Glu Thr His Gly Leu
                   545                 550                 555
   Leu Tyr Asn Leu Tyr Ala Asp Arg Glu Leu Gly Leu Asn Leu Val Pro
               560                 565                 570
   Gln Ser Val Tyr Asp Met Gln Asn Thr Phe Tyr Pro Thr Val Lys Glu
           575                 580                 585
   Lys Tyr Gly Val Pro Leu Asp Thr Arg His Val Tyr Thr Lys Ala Asp
       590                 595                 600
   Trp Glu Leu Phe Thr Ala Ala Val Ala Ser Glu Ser Val Arg Asp Met
   605                 610                 615                 620
   Phe His Gln Ala Leu Ala Thr Trp Ile Asn Glu Thr Pro Thr Asn Arg
                   625                 630                 635
   Ala Phe Thr Asp Leu Tyr Asp Thr Gln Thr Gly Asn Tyr Pro Ala Gly
               640                 645                 650
   Ile Thr Phe Ile Ala Arg Pro Val Met Gly Gly Ala Phe Ala Leu Leu
           655                 660                 665
   Ile Leu
       670
<210> 19
<211> 27
   <212> DNA
   <213> Artificial Sequence
<220>
   <223> Description of Artificial Sequence:primer
<400> 19
   gacgaccaag atggtctgag ctaccagt 27
<210> 20
   <211> 30
   <212> DNA
   <213> Artificial Sequence
<220>
   <223> Description of Artificial Sequence:primer
<400> 20
   gcaaagtcat ccgtagagat ctggttcgcc 30
<210> 21
   <211> 6191
   <212> DNA
   <213> Aspergillus nidulans
<220>
   <221> CDS
   <222> join (1807..2000, 2061..2353, 2412..2854, 2915..3498, 3554..3756, 3 806..3949, 3996..4131, 4180..4246)
<220>
   <221> intron
   <222> (2001)..(2060)
<220>
   <221> intron
   <222> (2354)..(2411)
<220>
   <221> intron
   <222> (2855)..(2914)
<220>
   <221> intron
   <222> (3499)..(3553)
<220>
   <221> intron
   <222> (3757)..(3805)
<220>
   <221> intron
   <222> (3950)..(3995)
<220>
   <221> intron
   <222> (4132)..(4179)
<220>
   <221> sig_peptide
   <222> (1807)..(1863)
<220>
   <221> mat_peptide
   <222> join(1864..2000, 2061..2353, 2412..2854, 2915..3498, 3554..3756, 3 806..3949, 3996..4131, 4180..4246)
<400> 21
<210> 22
   <211> 688
   <212> PRT
   <213> Aspergillus nidulans
<400> 22
   Met Arg Thr Phe Leu Leu Gly Ile Leu Cys Ala Pro Leu Ala Ile Leu
   -19 -15 -10 -5
   Thr Gly Ala Ala Ser Thr Phe Ser Pro Ala Arg Pro Pro Ala Leu Pro
   1 5 10
   Leu Ala Val Lys Ser Pro Tyr Leu Ser Thr Trp Leu Pro Ala Gly Lys
   15 20 25
   Asp Gly Gly Asn Gly Gly Tyr Leu Ala Gly Glu Trp Pro Ala Phe Trp
   30 35 40 45
   Glu Gly Gln Ile Thr Gly Trp Ala Gly Leu Ile Arg Val Asp Gly Gln
   50 55 60
   Val Tyr Thr Trp Met Gly Leu Pro Gly Ser Ala Thr Val Asn Gln Thr
   65 70 75
   Ala Tyr Glu Tyr Thr Ser Thr Lys Ser Ile Phe Thr Met His Ile Gly
   80 85 90
   Asp Met Val Glu Met Lys Ile Thr Phe Leu Ser Pro Ile Thr Pro Asn
   95 100 105
   Asp Leu Arg Arg Gln Ser Leu Val Phe Ser Tyr Leu Asp Val Ser Val
   110 115 120 125
   Thr Ser Leu Asp Gly Gln Ser His Ser Val Gln Val Tyr Ala Asp Ile
   130 135 140
   Ser Ala Glu Phe Ala Ser Gly Asp Arg Ser Ala Ile Ala Gln Trp Asn
   145 150 155
   Tyr Gly Val Thr Ser Asp Gly Val Ala Tyr His Lys Ile Tyr Arg Gln
   160 165 170
   Thr Pro Leu Leu Phe Ser Glu His Arg Asp Gln Ala Glu Trp Gly Asp
   175 180 185
   Trp Tyr Trp Ala Thr Asp Asn Val Ala Gly Leu Thr Tyr Gln Ala Gly
   190 195 200 205
   Pro Asp Val Asp Val Arg Glu Ala Phe Ala Arg Asn Gly Lys Leu Thr
   210 215 220
   Asn Asn Asn Asp Val Asn Tyr Arg Ala Ile Ser Asn Asn Trp Pro Val
   225 230 235
   Phe Gly Phe Ala His Asp Leu Gly Ser Ile Ser Ser Ser Thr Lys Val
   240 245 250
   Leu Phe Ser Ile Gly Leu Thr Gln Arg Glu Ala Ile Gln Tyr Ser Gly
   255 260 265
   Asn Ser Ser Thr Leu Ser Pro Leu Pro Ala Leu Trp Thr Ser Tyr Phe
   270 275 280 285
   Ser Thr Ala Leu Asp Ala Leu Asp Phe Phe His His Asp Tyr Gln Lys
   290 295 300
   Ser Asn Ser Leu Ser Ser Asp Leu Asp Arg Arg Ile Ala Gln Asp Ser
   305 310 315
   Val Ala Ala Ala Gly His Asp Tyr Leu Thr Ile Thr Ser Leu Ser Ile
   320 325 330
   Arg Gln Ala Phe Ala Ala Thr Gln Leu Cys Gly Pro Ala Asn Asp Pro
   335 340 345
   Tyr Leu Phe Met Lys Glu Ile Ser Ser Asn Gly Asn Met Asn Thr Val
   350 355 360 365
   Asp Val Ile Phe Pro Ala His Pro Val Phe Leu Tyr Thr Asn Pro Ala
   370 375 380
   Leu Leu Lys Tyr Leu Leu Arg Pro His Leu Glu Ile Gln Glu Ser Gly
   385 390 395
   Asn Tyr Pro Asn Ser Tyr Ala Met His Asp Ile Gly Ala His Tyr Pro
   400 405 410
   Asn Ala Thr Gly His Pro Asp Gly Asn Asp Glu Pro Met Pro Leu Glu
   415 420 425
   Glu Cys Gly Asn Met Val Ile Met Ala Leu Ala Tyr Ala Gln Lys Ala
   430 435 440 445
   Gly Asp Thr Ala Tyr Leu Glu Ser His Tyr Thr Ile Leu Arg Arg Trp
   450 455 460
   Thr Asp Tyr Leu Ile Glu Asp Ser Leu Tyr Pro Ala Asn Gln Ile Ser
   465 470 475
   Thr Asp Asp Phe Ala Gly Pro Leu Ala Asn Gln Thr Asn Leu Ala Leu
   480 485 490
   Lys Gly Ile Ile Gly Ile Glu Ala Met Ser Val Ile Ala Ser Leu Thr
   495 500 505
   Gly Asp Ser Asp Asp Lys Met Asn Leu Thr Asn Tyr Ala His Asp Tyr
   510 515 520 525
   Ile Glu Lys Trp Leu Ile Leu Gly Ile Ala Arg Asn Thr Thr Tyr Pro
   530 535 540
   His Thr Thr Leu Ser Tyr Gly Ser Asn Glu Ser His Gly Leu Leu Tyr
   545 550 555
   Asn Leu Tyr Ala Asp Arg Glu Leu Gly Leu Asn Leu Val Pro Gln Ser
   560 565 570
   Val Tyr Asp Met Gln Ser Asn Phe Tyr Pro Thr Ile Lys Gly Gln Tyr
   575 580 585
   Gly Val Pro Leu Asp Thr Arg His Gln Tyr Thr Lys Gly Asp Trp Glu
   590 595 600 605
   Leu Phe Thr Ala Ala Val Ala Ser Val Ser Thr Arg Asp Met Phe Ile
   610 615 620
   Lys Leu Leu Ala Gln Trp Ile Asn Glu Thr Pro Thr Asn Arg Pro Leu
   625 630 635
   Thr Asp Leu Tyr Asp Thr Val Thr Gly Asp Tyr Pro Pro Val Val Phe
   640 645 650
   Ile Ala Arg Pro Val Met Gly Ala Ala Phe Thr Leu Leu Leu Leu Asp
   655 660 665
<210> 23
   <211> 27
   <212> DNA
   <213> Artificial Sequence
<220>
   <223> Description of Artificial Sequence:primer
<400> 23
   gcttcataat tctcctgttg ttgagtc 27
<210> 24
   <211> 30
   <212> DNA
   <213> Artificial Sequence
<220>
   <223> Description of Artificial Sequence:primer
<400> 24
   ggctataact gatgctatat actaccacac 30
<210> 25
   <211> 2298
   <212> DNA
   <213> Aspergillus nidulans
<220>
   <221> CDS
   <222> (88)..(2151)
   <220>
   <221> mat_peptide
   <222> (145)..(2151)
<400> 25
   gcttcataat tctcctgttg ttgagtcttt taaacggggc ctattagaag ggggttgcac 60
   ggctgaggat cacctaccca gatcaat atg cgt act ttt cta cta ggc atc ctg 114
                                 Met Arg Thr Phe Leu Leu Gly Ile Leu
                                                 -15
   tgc gca ccc ctg gct atc ctt aca gga gcc gca tcg act ttt tct cca   162
   Cys Ala Pro Leu Ala Ile Leu Thr Gly Ala Ala Ser Thr Phe Ser Pro
   -10                 -5                  -1   1               5
   gca cgc cct ccg gct ctt cct cta gcg gtc aaa tct ccg tac ttg agt   210
   Ala Arg Pro Pro Ala Leu Pro Leu Ala Val Lys Ser Pro Tyr Leu Ser
               10                  15                  20
   act tgg ctg ccg gcg ggg aaa gac ggc ggc aat gga ggc tac ctt gca   258
   Thr Trp Leu Pro Ala Gly Lys Asp Gly Gly Asn Gly Gly Tyr Leu Ala
           25                  30                  35
   ggg gaa tgg cca gcg ttc tgg gaa ggc caa ata aca gga tgg gct ggc   306
   Gly Glu Trp Pro Ala Phe Trp Glu Gly Gln Ile Thr Gly Trp Ala Gly
       40                  45                  50
   ctt att cgt gtg gat ggc cag gtc tat aca tgg atg ggc ctt cca ggc   354
   Leu Ile Arg Val Asp Gly Gln Val Tyr Thr Trp Met Gly Leu Pro Gly
    55                  60                  65                  70
   tca gcc act gtg aac cag act gcg tat gag tac act tca acg aag agc   402
   Ser Ala Thr Val Asn Gln Thr Ala Tyr Glu Tyr Thr Ser Thr Lys Ser
                    75                  80                  85
   att ttc acc atg cat att ggt gat atg gta gag atg aag ata acc ttc   450
   Ile Phe Thr Met His Ile Gly Asp Met Val Glu Met Lys Ile Thr Phe
                90                  95                  100
   ctt tca cca att aca ccg aat gat ctt cga cgg cag tcc cta gtg ttt   498
   Leu Ser Pro Ile Thr Pro Asn Asp Leu Arg Arg Gln Ser Leu Val Phe
           105                 110                 115
   tcg tat ctt gac gtg agt gtc acc tca ctc gac ggc cag tcc cac agt   546
   Ser Tyr Leu Asp Val Ser Val Thr Ser Leu Asp Gly Gln Ser His Ser
       120                 125                 130
   gta cag gtg tac gct gac ata tca gct gaa ttt gcg tct ggc gac cgt   594
   Val Gln Val Tyr Ala Asp Ile Ser Ala Glu Phe Ala Ser Gly Asp Arg
   135                 140                 145                 150
   tcc gcc ata gca caa tgg aac tat ggt gtt acc agt gac ggc gta gcc   642
   Ser Ala Ile Ala Gln Trp Asn Tyr Gly Val Thr Ser Asp Gly Val Ala
                   155                 160                 165
   tat cat aag atc tat cgc cag acg ccg ctc cta ttc tct gag cat aga   690
   Tyr His Lys Ile Tyr Arg Gln Thr Pro Leu Leu Phe Ser Glu His Arg
               170                 175                 180
   gac caa gct gaa tgg ggt gat tgg tac tgg gca act gac aat gta gca   738
   Asp Gln Ala Glu Trp Gly Asp Trp Tyr Trp Ala Thr Asp Asn Val Ala
           185                 190                 195
   gga ctc act tac cag gct ggt cca gat gtt gat gtc cgg gaa gct ttt   786
   Gly Leu Thr Tyr Gln Ala Gly Pro Asp Val Asp Val Arg Glu Ala Phe
       200                 205                 210
   gcg cgc aat gga aag cta acc aat aac aac gac gtc aac tac aga gct   834
   Ala Arg Asn Gly Lys Leu Thr Asn Asn Asn Asp Val Asn Tyr Arg Ala
   215                 220                 225                 230
   atc tcc aac aac tgg ccg gtg ttt ggt ttt gcc cat gac ctt ggg tct   882
   Ile Ser Asn Asn Trp Pro Val Phe Gly Phe Ala His Asp Leu Gly Ser
                   235                 240                 245
   atc agc tct tct act aag gtg ctt ttc tca ata ggg cta acc cag cga   930
   Ile Ser Ser Ser Thr Lys Val Leu Phe Ser Ile Gly Leu Thr Gln Arg
               250                 255                 260
   gag gca atc cag tat agc ggg aac tct tcc acc ctt tct cct ttg cct   978
   Glu Ala Ile Gln Tyr Ser Gly Asn Ser Ser Thr Leu Ser Pro Leu Pro
           265                 270                 275
   gct ctg tgg acg agc tat ttc agc act gcc ttg gat gcc ctt gac ttc   1026
   Ala Leu Trp Thr Ser Tyr Phe Ser Thr Ala Leu Asp Ala Leu Asp Phe
       280                 285                 290
   ttc cac cat gat tat cag aag tca aac tct ctt tct tca gat ctt gat   1074
   Phe His His Asp Tyr Gln Lys Ser Asn Ser Leu Ser Ser Asp Leu Asp
   295                 300                 305                 310
   cgg cga att gca caa gat tcc gtt gcc gct gcc ggt cac gac tac ctt    1122
   Arg Arg Ile Ala Gln Asp Ser Val Ala Ala Ala Gly His Asp Tyr Leu
                   315                 320                 325
   acc att aca tcc ctc agc att cgt caa gct ttc gct gca acc cag ctg    1170
   Thr Ile Thr Ser Leu Ser Ile Arg Gln Ala Phe Ala Ala Thr Gln Leu
               330                 335                 340
   tgt ggg cca gca aat gat ccg tat ctc ttt atg aaa gaa atc tcc tcc    1218
   Cys Gly Pro Ala Asn Asp Pro Tyr Leu Phe Met Lys Glu Ile Ser Ser
           345                 350                 355
   aac ggc aac atg aac acg gta gat gtg atc ttc cct gct cat ccc gtc    1266
   Asn Gly Asn Met Asn Thr Val Asp Val Ile Phe Pro Ala His Pro Val
       360                 365                 370
   ttc tta tac aca aac cca gca ctg ctt aaa tat ctc ctg cgc cca cat    1314
   Phe Leu Tyr Thr Asn Pro Ala Leu Leu Lys Tyr Leu Leu Arg Pro His
   375                 380                 385                 390
   ttg gag atc cag gag tct gga aat tac ccc aac tcc tat gct atg cat    1362
   Leu Glu Ile Gln Glu Ser Gly Asn Tyr Pro Asn Ser Tyr Ala Met His
                   395                 400                 405
   gat atc ggt gct cat tac cct aac gct aca ggc cat ccg gat ggc aat    1410
   Asp Ile Gly Ala His Tyr Pro Asn Ala Thr Gly His Pro Asp Gly Asn
               410                 415                 420
   gac gag cca atg ccg ttg gag gag tgc ggt aat atg gtg atc atg gct    1458
   Asp Glu Pro Met Pro Leu Glu Glu Cys Gly Asn Met Val Ile Met Ala
           425                 430                 435
   cta gca tat gcg cag aag gcc ggg gac aca gcg tac ctg gaa agc cac    1506
   Leu Ala Tyr Ala Gln Lys Ala Gly Asp Thr Ala Tyr Leu Glu Ser His
       440                 445                 450
   tac aca ata ctg aga cgt tgg acg gac tac ttg atc gaa gat tct ctt    1554
   Tyr Thr Ile Leu Arg Arg Trp Thr Asp Tyr Leu Ile Glu Asp Ser Leu
   455                 460                 465                 470
   tat ccg gcg aac caa ata tcg aca gat gat ttc gca ggt cca ttg gca    1602
   Tyr Pro Ala Asn Gln Ile Ser Thr Asp Asp Phe Ala Gly Pro Leu Ala
                   475                 480                 485
   aat caa acc aac ctc gcc ctg aag gga atc atc ggt atc gag gcc atg    1650
   Asn Gln Thr Asn Leu Ala Leu Lys Gly Ile Ile Gly Ile Glu Ala Met
               490                 495                 500
   tct gtc atc gct agc ctg aca gga gac tct gat gat aag atg aat ctc    1698
   Ser Val Ile Ala Ser Leu Thr Gly Asp Ser Asp Asp Lys Met Asn Leu
           505                 510                 515
   acc aat tac gcc cac gat tac atc gaa aaa tgg ctg att ttg gga att    1746
   Thr Asn Tyr Ala His Asp Tyr Ile Glu Lys Trp Leu Ile Leu Gly Ile
       520                 525                 530
   gca cgt aac aca acg tat ccg cat aca aca ttg tcg tac gga tca aac    1794
   Ala Arg Asn Thr Thr Tyr Pro His Thr Thr Leu Ser Tyr Gly Ser Asn
   535                 540                 545                 550
   gag tct cat gga ctc ctg tac aac ctt tac gca gat cgc gag cta ggc    1842
   Glu Ser His Gly Leu Leu Tyr Asn Leu Tyr Ala Asp Arg Glu Leu Gly
                   555                 560                 565
   ttg aac ctg gtc ccg caa tct gtg tat gac atg cag agc aac ttc tac    1890
   Leu Asn Leu Val Pro Gln Ser Val Tyr Asp Met Gln Ser Asn Phe Tyr
               570                 575                 580
   ccg aca atc aag ggt caa tac gga gtg cca ctg gat acc cgc cat caa    1938
   Pro Thr Ile Lys Gly Gln Tyr Gly Val Pro Leu Asp Thr Arg His Gln
           585                 590                 595
   tac acg aaa ggt gat tgg gag ctc ttc acg gcg gca gtc gca tca gta    1986
   Tyr Thr Lys Gly Asp Trp Glu Leu Phe Thr Ala Ala Val Ala Ser Val
       600                 605                 610
   agc aca cga gac atg ttc atc aaa cta ctt gcc cag tgg ata aac gag    2034
   Ser Thr Arg Asp Met Phe Ile Lys Leu Leu Ala Gln Trp Ile Asn Glu
   615                 620                 625                 630
   acg cca acg aac cgt cca ctt acg gat ctc tat gac act gta acc gga    2082
   Thr Pro Thr Asn Arg Pro Leu Thr Asp Leu Tyr Asp Thr Val Thr Gly
                   635                 640                 645
   gac tac cca ccg gta gtt ttc atc gct cga cct gtc atg ggc gcg gca    2130
   Asp Tyr Pro Pro Val Val Phe Ile Ala Arg Pro Val Met Gly Ala Ala
               650                 655                 660
   ttc act ttg ttg ctt ctc gac tgaaattctt tccaggtaca tacatgcttt       2181
   Phe Thr Leu Leu Leu Leu Asp
           665
   atcaggacaa aagagcggct aggcggtcca ttcccagtat ttgtgttaga tgtgatatat 2241
   atggtcgtgt ttaagttgtg taatattgtg tggtagtata tagcatcagt tatagcc    2298
<210> 26
   <211> 688
   <212> PRT
   <213> Aspergillus nidulans
<400> 26
   Met Arg Thr Phe Leu Leu Gly Ile Leu Cys Ala Pro Leu Ala Ile Leu
                   -15                 -10                  -5
   Thr Gly Ala Ala Ser Thr Phe Ser Pro Ala Arg Pro Pro Ala Leu Pro
            -1   1               5                  10
   Leu Ala Val Lys Ser Pro Tyr Leu Ser Thr Trp Leu Pro Ala Gly Lys
        15                  20                  25
   Asp Gly Gly Asn Gly Gly Tyr Leu Ala Gly Glu Trp Pro Ala Phe Trp
   30 35 40 45
   Glu Gly Gln Ile Thr Gly Trp Ala Gly Leu Ile Arg Val Asp Gly Gln
   50 55 60
   Val Tyr Thr Trp Met Gly Leu Pro Gly Ser Ala Thr Val Asn Gln Thr
   65 70 75
   Ala Tyr Glu Tyr Thr Ser Thr Lys Ser Ile Phe Thr Met His Ile Gly
   80 85 90
   Asp Met Val Glu Met Lys Ile Thr Phe Leu Ser Pro Ile Thr Pro Asn
   95 100 105
   Asp Leu Arg Arg Gln Ser Leu Val Phe Ser Tyr Leu Asp Val Ser Val
   110 115 120 125
   Thr Ser Leu Asp Gly Gln Ser His Ser Val Gln Val Tyr Ala Asp Ile
   130 135 140
   Ser Ala Glu Phe Ala Ser Gly Asp Arg Ser Ala Ile Ala Gln Trp Asn
   145 150 155
   Tyr Gly Val Thr Ser Asp Gly Val Ala Tyr His Lys Ile Tyr Arg Gln
   160 165 170
   Thr Pro Leu Leu Phe Ser Glu His Arg Asp Gln Ala Glu Trp Gly Asp
   175 180 185
   Trp Tyr Trp Ala Thr Asp Asn Val Ala Gly Leu Thr Tyr Gln Ala Gly
   190 195 200 205
   Pro Asp Val Asp Val Arg Glu Ala Phe Ala Arg Asn Gly Lys Leu Thr
   210 215 220
   Asn Asn Asn Asp Val Asn Tyr Arg Ala Ile Ser Asn Asn Trp Pro Val
   225 230 235
   Phe Gly Phe Ala His Asp Leu Gly Ser Ile Ser Ser Ser Thr Lys Val
   240 245 250
   Leu Phe Ser Ile Gly Leu Thr Gln Arg Glu Ala Ile Gln Tyr Ser Gly
   255 260 265
   Asn Ser Ser Thr Leu Ser Pro Leu Pro Ala Leu Trp Thr Ser Tyr Phe
   270 275 280 285
   Ser Thr Ala Leu Asp Ala Leu Asp Phe Phe His His Asp Tyr Gln Lys
   290 295 300
   Ser Asn Ser Leu Ser Ser Asp Leu Asp Arg Arg Ile Ala Gln Asp Ser
   305 310 315
   Val Ala Ala Ala Gly His Asp Tyr Leu Thr Ile Thr Ser Leu Ser Ile
   320 325 330
   Arg Gln Ala Phe Ala Ala Thr Gln Leu Cys Gly Pro Ala Asn Asp Pro
   335 340 345
   Tyr Leu Phe Met Lys Glu Ile Ser Ser Asn Gly Asn Met Asn Thr Val
   350 355 360 365
   Asp Val Ile Phe Pro Ala His Pro Val Phe Leu Tyr Thr Asn Pro Ala
   370 375 380
   Leu Leu Lys Tyr Leu Leu Arg Pro His Leu Glu Ile Gln Glu Ser Gly
   385 390 395
   Asn Tyr Pro Asn Ser Tyr Ala Met His Asp Ile Gly Ala His Tyr Pro
   400 405 410
   Asn Ala Thr Gly His Pro Asp Gly Asn Asp Glu Pro Met Pro Leu Glu
   415 420 425
   Glu Cys Gly Asn Met Val Ile Met Ala Leu Ala Tyr Ala Gln Lys Ala
   430 435 440 445
   Gly Asp Thr Ala Tyr Leu Glu Ser His Tyr Thr Ile Leu Arg Arg Trp
   450 455 460
   Thr Asp Tyr Leu Ile Glu Asp Ser Leu Tyr Pro Ala Asn Gln Ile Ser
   465 470 475
   Thr Asp Asp Phe Ala Gly Pro Leu Ala Asn Gln Thr Asn Leu Ala Leu
   480 485 490
   Lys Gly Ile Ile Gly Ile Glu Ala Met Ser Val Ile Ala Ser Leu Thr
   495 500 505
   Gly Asp Ser Asp Asp Lys Met Asn Leu Thr Asn Tyr Ala His Asp Tyr
   510 515 520 525
   Ile Glu Lys Trp Leu Ile Leu Gly Ile Ala Arg Asn Thr Thr Tyr Pro
   530 535 540
   His Thr Thr Leu Ser Tyr Gly Ser Asn Glu Ser His Gly Leu Leu Tyr
   545 550 555
   Asn Leu Tyr Ala Asp Arg Glu Leu Gly Leu Asn Leu Val Pro Gln Ser
   560 565 570
   Val Tyr Asp Met Gln Ser Asn Phe Tyr Pro Thr Ile Lys Gly Gln Tyr
   575 580 585
   Gly Val Pro Leu Asp Thr Arg His Gln Tyr Thr Lys Gly Asp Trp Glu
   590 595 600 605
   Leu Phe Thr Ala Ala Val Ala Ser Val Ser Thr Arg Asp Met Phe Ile
   610 615 620
   Lys Leu Leu Ala Gln Trp Ile Asn Glu Thr Pro Thr Asn Arg Pro Leu
   625 630 635
   Thr Asp Leu Tyr Asp Thr Val Thr Gly Asp Tyr Pro Pro Val Val Phe
   640 645 650
   Ile Ala Arg Pro Val Met Gly Ala Ala Phe Thr Leu Leu Leu Leu Asp
   655 660 665